(19) Europäisches Patentamt
European Patent Office
Office européen des brevets

(11) EP 2 039 347 A1

# (12) EUROPEAN PATENT APPLICATION

published in accordance with Art. 153(4) EPC

(43) Date of publication:
**25.03.2009 Bulletin 2009/13**

(21) Application number: **07767893.6**

(22) Date of filing: **29.06.2007**

(51) Int Cl.:
***A61K 8/898*** (2006.01) ***A61K 8/899*** (2006.01)
***A61Q 5/00*** (2006.01) ***A61Q 5/06*** (2006.01)
***C08F 290/06*** (2006.01) ***C08G 18/65*** (2006.01)

(86) International application number:
**PCT/JP2007/063106**

(87) International publication number:
**WO 2008/004502 (10.01.2008 Gazette 2008/02)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **03.07.2006 JP 2006183144**

(71) Applicants:
- **Shiseido Company, Limited**
**Chuo-ku**
**Tokyo 104-8010 (JP)**
- **KONISHI CO., LTD.**
**Osaka-shi,**
**Osaka-fu 541-0045 (JP)**

(72) Inventors:
- **SHIMIZU, Hideki**
**Yokohama-shi**
**Kanagawa 224-8558 (JP)**
- **WATANABE, Tomoko**
**Yokohama-shi**
**Kanagawa 224-8558 (JP)**
- **KAWASOE, Tomoyuki**
**Yokohama-shi**
**Kanagawa 224-8558 (JP)**
- **FUJIYAMA, Taizo**
**Yokohama-shi**
**Kanagawa 224-8558 (JP)**
- **ORIGUCHI, Toshiki**
**Osaka-shi**
**Osaka 538-0053 (JP)**
- **OGAWA, Shintaro**
**Osaka-shi**
**Osaka 538-0053 (JP)**
- **NAKAYAMA, Yoshitaka**
**Osaka-shi**
**Osaka 538-0053 (JP)**

(74) Representative: **Grünecker, Kinkeldey,**
**Stockmair & Schwanhäusser**
**Anwaltssozietät**
**Leopoldstrasse 4**
**80802 München (DE)**

## (54) COSMETIC HAIR PREPARATION

(57) Provided is a hair cosmetic that can exhibit hair styling capability, natural finishing capability, and hair style retentivity respectively at superior levels.

The hair cosmetic contains a urethane-modified acrylic polymer having a structure in which a urethane polymer chain is bonded to an acrylic polymer chain through a linkage segment having a silicon-oxygen bond, in which the acrylic polymer chain is a residue of an acrylic polymer derived at least from an acrylic monomer (A) and a compound (B1) and/or a compound (B2), where the compound (B1) has a hydrolyzable silicon-containing group and an ethylenically unsaturated bond-containing group, and the compound (B2) has a hydrolyzable silicon-containing group and a mercapto group, and the urethane polymer chain is a residue of a urethane polymer (C) having a hydrolyzable silicon-containing group. The linkage segment in the urethane-modified acrylic polymer is preferably a silicone polymer chain.

EP 2 039 347 A1

## Description

### Technical Field

The present invention relates to hair cosmetics. More specifically, it relates to hair cosmetics that enable the hair to set to a predetermined hair style (hair styling capability), impart a natural-feeling finish to the set hair (natural finishing capability), and retain the set hair style even under severe conditions of high humidity (hair style retentivity). The hair cosmetics can exhibit these properties at superior levels.

### Background Art

Known commercially available hair cosmetics include, for example, hard-type hair styling agents that enable the hair to be set in a hard or firm hair style and tightly retain the set hair style; and soft-type hair styling agents that impart a smooth touch and a soft finish to the hair with less coarse/stiff feeling. Among such hair styling agents, hard-type hair styling agents mostly use film-formable polymer components such as polyvinylpyrrolidones, sodium polyacrylates, and polyvinylpyrrolidone-poly(vinyl acetate) copolymers (see Patent Document 1). These hard-type hair styling agents exhibit high hair styling capability through gluing hairs by the action of the film-formable polymer components.
On the other hand, soft-type hair styling agents mostly use waxes such as beeswax, lanolin, and candelilla wax (see Patent Document 2). Such waxes are lipids that are solid or semi-solid at ordinary temperature, and hair styling agents using these waxes give a soft finish with less coarse/stiff feeling.
There is known, as a urethane-modified acrylic polymer, a vinyl-urethane copolymer having a vinyl polymer chain and a urethane polymer chain, in which the vinyl polymer chain and the urethane polymer chain are bonded with each other through a linkage segment having a silicon-oxygen bond (see Patent Document 3). PCT International Publication No. WO 2005/054341 (WO 2005/054341 A1) discloses the use of this vinyl-urethane copolymer in a hair cosmetic.
Patent Document 1: Japanese Unexamined Patent Application Publication (JP-A) No. 2003-171244
Patent Document 2: JP-A No. 2000-72626
Patent Document 3: PCT International Publication No. WO 2005/054341

### Disclosure of Invention

### Problems to be Solved by the Invention

However, known hard-type hair styling agents are difficult to impart a natural finish to the set hair, because hairs set with these hair styling agents form strands and are stiff and coarse, through which a hand or comb not easily goes through; on the other hand, known soft-type hair styling agents hardly retain the set hair style for a long time and show strong stickiness to the hand or hair particularly under conditions at high humidity in the summer months. Thus, the known hair styling agents hardly exhibit hair styling capability, natural finishing capability, and hair style retentivity respectively at superior levels, respectively. The hair styling capability is a capability of setting the hair to a predetermined hair style; the natural finishing capability is a capability of imparting finish with natural feeling to the hair with no or substantially no coarse/stiff feeling; and the hair style retentivity is a capability of retaining the set hair style even under severe conditions of high humidity. Additionally, hair styling agents should have a capability of setting the hair with a pliable and smooth touch (smooth touch). Consequently, known commercially available hair styling agents are not always products responding to consumer demands.
Accordingly, an object of the present invention is to provide a hair cosmetic that exhibits hair styling capability, natural finishing capability, and hair style retentivity respectively at superior levels.
Another object of the present invention is to provide a hair cosmetic that develops a smooth touch at a superior level, in addition to the above properties.

### Means for Solving the Problems

After intensive investigations to achieve the objects, the present inventors found that use of a specific urethane-modified acrylic polymer and control of components of the urethane-modified acrylic polymer gives a hair cosmetic that has desired properties typified by hair styling capability, natural finishing capability to set the hair with a finish with natural feeling, and hair style retentivity to retain or keep the set hair style even under severe conditions of high humidity. Specifically, they found that, when an importance is placed on the hair styling capability and hair style retentivity, there can be obtained a hair cosmetic that exhibits further superior hair styling capability and hair style retentivity while having

natural finishing capability and smooth touch, and, when an importance is placed on the smooth finish, there can be obtained a hair cosmetic that exhibits superior natural finishing capability and smooth touch to impart a natural finish to the set hair with no or substantially no coarse/stiff feeling while keeping certain hair style retentivity. The present invention has been made based on these findings.

Specifically, the present invention provides a hair cosmetic which contains a urethane-modified acrylic polymer having a structure in which a urethane polymer chain is bonded to an acrylic polymer chain through a linkage segment containing a silicon-oxygen bond, in which
the acrylic polymer chain is a residue of an acrylic polymer derived at least from an acrylic monomer (A) and one or both of a compound (B1) and a compound (B2), where the compound (B1) has a hydrolyzable silicon-containing group and an ethylenically unsaturated bond-containing group, and the compound (B2) has a hydrolyzable silicon-containing group and a mercapto group; and
the urethane polymer chain is a residue of a urethane polymer (C) having a hydrolyzable silicon-containing group.

In the hair cosmetic according to the present invention, the urethane polymer (C) having a hydrolyzable silicon-containing group is preferably an alkoxysililated urethane polymer (C1) having a hydrophilic group. The alkoxysililated urethane polymer (C1) having a hydrophilic group is preferably a terminally alkoxysililated urethane polymer having a hydrophilic group which corresponds to a urethane polymer having a hydrophilic group, at least part of terminal isocyanate groups of the urethane polymer being alkoxysililated. An exemplary hydrophilic-group-containing alkoxysililated urethane polymers (C1) is a urethane polymer having a hydrophilic group and a terminal alkoxysilyl group, which is a reaction product among following Components (C1-a), (C1-b), (C1-c), and (C1-d):

(C1-a): a compound having two or more active-hydrogen-containing groups but having no hydrophilic group;
(C1-b): a compound having a hydrophilic group and two or more active-hydrogen-containing groups;
(C1-c): a polyisocyanate compound; and
(C1-d): an alkoxysilane compound having an active-hydrogen-containing group.

The alkoxysilane compound (C1-d) having an active-hydrogen-containing group is preferably an amino-containing alkoxysilane compound (C1-d1) that contains an amino group as the active-hydrogen-containing group.

The linkage segment in the hair cosmetic according to the present invention is preferably a silicone polymer chain. The silicone polymer chain preferably comprises a hydrolyzable silicon-containing group and an ethylenically unsaturated bond-containing group of the compound (B1) and/or a hydrolyzable silicon-containing group of the compound (B2); a hydrolyzable silicon-containing group of the urethane polymer (C); and a silane compound (D) having a hydrolyzable silicon-containing group.

The silane compound (D) having a hydrolyzable silicon-containing group is preferably an alkoxy-containing silane compound (D1). When an importance is placed on the smooth finish, the alkoxy-containing silane compound (D1) preferably contains at least a dialkoxysilyl-containing silane compound (D1-1). When an importance is placed on the hair styling capability and hair style retentivity, the alkoxy-containing silane compound (D1) preferably contains at least a trialkoxysilyl-containing silane compound (D1-2). The alkoxy-containing silane compound (D1) may contain both a dialkoxysilyl-containing silane compound (D1-1) and a trialkoxysilyl-containing silane compound (D1-2).

The hair cosmetic according to the present invention preferably contains 0.1 to 10 percent by mass of the urethane-modified acrylic polymer.

As used herein the term "hair" means and includes scalp hairs, cilia, eyebrow, and other body hairs that grow allover the human body, except for body hairs that grow on palms, soles of the foot, lips, nipples, and mucosal areas such as and genital areas. Accordingly, hair cosmetics according to the present invention are cosmetics that are applied typically to scalp hairs, cilia, and supercilia. Specifically, the hair cosmetics includes scalp hair cosmetics such as hair styling sprays, hair styling gels, hair waxes, and hair styling mousses; cilia cosmetics such as mascaras; and supercilia cosmetics.

Advantages

The hair cosmetics according to the present invention have the above configurations, can thereby exhibit hair styling capability (hair dressing capability), natural finishing capability, and hair style retentivity respectively at superior levels, and can develop a smooth touch. The hair cosmetics, if used, allow the hair to set to a predetermined hair style easily, retain the set hair style for a long time even in the summer months, and impart a natural feeling to the set hair. Additionally, the hair cosmetics impart a pliable and smooth touch to the set hair. Best Mode for Carrying Out the Invention

Hair cosmetics according to the present invention each include a urethane-modified acrylic polymer having a structure in which a urethane polymer chain is bonded to an acrylic polymer chain through a linkage segment having a silicon-oxygen bond (siloxane bond; Si-O bond) (hereinafter also referred to as "Si-O bond-containing linkage segment"), in which the acrylic polymer chain is a residue of an acrylic polymer derived at least from an acrylic monomer (A) and

one or both of a compound (B1) and a compound (B2), where the compound (B1) has a hydrolyzable silicon-containing group and an ethylenically unsaturated bond-containing group, and the compound (B2) has a hydrolyzable silicon-containing group and a mercapto group; and

the urethane polymer chain is a residue of a urethane polymer (C) having a hydrolyzable silicon-containing group.

Exemplary urethane-modified acrylic polymers include, of the vinyl-urethane copolymers disclosed in the description of PCT International Publication No. WO 2005/054341 A1 (i.e., vinyl-urethane copolymers each having a structure in which a vinyl polymer chain is bonded to a urethane polymer chain through a linkage segment having a silicon-oxygen bond), those in which the vinyl polymer chain is an acrylic polymer chain of a residue of an acrylic polymer derived at least from an acrylic monomer and one or both of a compound having a hydrolyzable silicon-containing group and an ethylenically unsaturated bond-containing group, and a compound having a hydrolyzable silicon-containing group and a mercapto group; and the urethane polymer chain is a urethane polymer chain of a residue of a urethane polymer having a hydrolyzable silicon-containing group.

Specifically, the hair cosmetics according to the present invention do not merely employ, as film-formable polymer components, the vinyl-urethane copolymers disclosed in WO 2005/054341 A1 but selectively employ, from among the vinyl-urethane copolymers, urethane-modified acrylic polymers whose vinyl polymer chains are limited to specific acrylic polymer chains and whose urethane copolymer chains are limited to specific urethane polymer chains. The urethane-modified acrylic polymers are acrylic polymers which are modified with urethane polymers.

Thus, the hair cosmetics according to the present invention contain specific urethane-modified acrylic polymers and can provide desirable hair cosmetics corresponding to demands, by regulating components of the urethane-modified acrylic copolymers. Typically, they can provide hair cosmetics that exhibit significantly superior hair styling capability and hair style retentivity; and hair cosmetics that enable finish with a natural feeling but with no or substantially no coarse/stiff feeling and give a pliable and smooth touch.

[Urethane-Modified Acrylic Polymers]

Urethane-modified acrylic polymers herein each have a structure in which the acrylic polymer chain and the urethane polymer chain are bonded with each other through a Si-O bond-containing linkage segment. Specifically, exemplary urethane-modified acrylic polymers include vinyl-urethane copolymers of Formula (1) ["$X^1$-$(W^1)_{u1}$-$X^3$-$(W^2)_{u2}$-$X^2$"] listed as specific examples of vinyl-urethane copolymers in WO 2005/054341 A1, in which $X^1$ is a urethane polymer chain as a residue of a urethane polymer (C) having a hydrolyzable silicon-containing group; and $X^2$ is an acrylic polymer chain as a residue of an acrylic polymer derived at least from an acrylic monomer (A) and one or both of a compound (B1) having a hydrolyzable silicon-containing group and an ethylenically unsaturated bond-containing group and a compound (B2) having a hydrolyzable silicon-containing group and a mercapto group. In Formula (1), $X^3$ is a Si-O bond-containing linkage segment; $W^1$ is a bivalent organic group; $W^2$ is a bivalent organic group; "$u^1$" is 0 or 1; and "$u^2$" is 0 or 1. The Si-O bond-containing linkage segment as $X^3$, the bivalent organic group as $W^1$, and the bivalent organic group as $W^2$ are as in WO 2005/054341 A1.

The Si-O bond-containing linkage segment may be any of low-molecular-weight (or low-molecular type) Si-O bond-containing linkage segments and high-molecular-weight (or high-molecular type) Si-O bond-containing linkage segments, as long as having a Si-O bond, as described in WO 2005/054341 A1. High-molecular-weight Si-O bond-containing linkage segments can act as silicone polymer chains; and low-molecular-weight Si-O bond-containing linkage segments can act as silicone segments. These Si-O bond-containing linkage segments typically high-molecular-weight Si-O bond-containing linkage segments may form a reticular structure. When the Si-O bond-containing linkage segment forms a high-molecular-weight Si-O bond-containing linkage segment (silicone polymer chain), two or more acrylic polymer chains and/or two or more urethane polymer chains may be bonded to one silicone polymer chain. Conversely, two or more silicone polymer chains may be bonded to one acrylic polymer chain and/or one urethane polymer chain. Not all the silicone polymer chains in a urethane-modified acrylic polymer may be bonded to one or more acrylic polymer chains and one or more urethane polymer chains.

As has been described, the urethane-modified acrylic polymers may further include a bivalent organic group that is positioned between an acrylic polymer chain and a Si-O bond-containing linkage segment and/or between a urethane polymer chain and a Si-O bond-containing linkage segment, as long as the acrylic polymer chain and the urethane polymer chain are bonded with each other through the Si-O bond-containing linkage segment.

The acrylic polymer chain is a residue of an acrylic polymer derived at least from an acrylic monomer (A) and one or both of a compound (B1) having a hydrolyzable silicon-containing group and an ethylenically unsaturated bond-containing group and a compound (B2) having a hydrolyzable silicon-containing group and a mercapto group. Accordingly, the acrylic polymer chain is basically composed of an acrylic polymer of an acrylic monomer (A) with one or more monomer components that are copolymerizable with the acrylic monomer (A). By way of example, the acrylic polymer chain herein is basically composed of an acrylic polymer moiety in the principal chain or backbone of the acrylic polymer. The urethane polymer chain is a residue of a urethane.polymer (C) having a hydrolyzable silicon-containing group and

is basically composed of an urethane polymer in the urethane polymer (C) having a hydrolyzable silicon-containing group. Typically, the urethane polymer chain is composed of a urethane polymer moiety of the principal chain or backbone of the urethane polymer. The silicone polymer chain is preferably composed of the hydrolyzable silicon-containing group of the compound (B1) and/or the hydrolyzable silicon-containing group of the compound (B1); the hydrolyzable silicon-containing group of the urethane polymer (C); and a silane compound (D) having a hydrolyzable silicon-containing group. The silicone polymer chain is therefore preferably basically composed of a polymer (silicone polymer) of the silane compound (D) having a hydrolyzable silicon-containing group. Typically, the silicone polymer chain is preferably basically composed of a silicone polymer moiety of the principal chain or backbone of the silicone polymer.

Accordingly, exemplary urethane-modified acrylic polymers include (1) urethane-modified acrylic polymers each having two different polymer chains, i.e., an acrylic polymer chain and a urethane polymer chain, as polymer chains (hereinafter also referred to as "urethane-modified acrylic binary copolymers") and (2) urethane-modified acrylic polymers each having three different polymer chains, i.e., an acrylic polymer chain, a silicone polymer chain, and a urethane polymer chain, as polymer chains (hereinafter also referred to as "urethane-modified acrylic ternary copolymers").

Exemplary urethane-modified acrylic ternary copolymers include vinyl-urethane copolymers of Formula (2) listed as examples of vinyl-urethane copolymers in WO 2005/054341 A1, in which $X^{1a}$ is a urethane polymer chain as a residue of a urethane polymer (C) having a hydrolyzable silicon-containing group; and $X^{2a}$ is an acrylic polymer chain as a residue of an acrylic polymer derived at least from an acrylic monomer (A) and one or both of a compound (B1) having a hydrolyzable silicon-containing group and an ethylenically unsaturated bond-containing group and a compound (B2) having a hydrolyzable silicon-containing group and a mercapto group. In Formula (2), $W^1$ is a bivalent organic group; $W^2$ is a bivalent organic group; "$u^1$" is 0 or 1; "$u^2$" is 0 or 1; and "$u^3$" is an integer of 1 or more.

(Acrylic Monomers (A))

Exemplary acrylic monomers (A) include acrylic esters and methacrylic esters [(meth)acrylic esters]. The (meth) acrylic esters include, for example, (meth)acrylic alkyl esters, (meth)acrylic cycloalkyl esters, and (meth)acrylic aryl esters. Specific examples of these are as listed in WO 2005/054341 A1. Each of different acrylic monomers (A) may be used alone or in combination. Preferred acrylic monomers (A) include (meth)acrylic alkyl esters. More specifically, preferred acrylic monomers (A) include methyl (meth)acrylates, ethyl (meth)acrylates, butyl (meth)acrylates, octyl (meth) acrylates, 2-ethylhexyl (meth)acrylates, isooctyl (meth)acrylates, nonyl (meth)acrylates, isononyl (meth)acrylates, dodecyl (meth)acrylates, octadecyl (meth)acrylates, and cyclohexyl (meth)acrylates.

One or more ethylenically unsaturated monomers that are copolymerizable with an acrylic monomer (A) (copolymerizable unsaturated monomers) may be used herein in combination with the acrylic monomer (A). Each of different copolymerizable unsaturated monomers may be used alone or in combination. Exemplary copolymerizable unsaturated monomers are those listed as copolymerizable unsaturated monomers in WO 2005/054341 A1. Exemplary preferred copolymerizable unsaturated monomers include carboxyl-containing monomers such as (meth)acrylic acids (acrylic acid and methacrylic acid); acid anhydride-containing monomers such as maleic anhydride and itaconic anhydride; hydroxyl-containing monomers such as hydroxyethyl (meth)acrylates; epoxy-containing monomers such as glycidyl (meth)acrylates; amino-containing monomers such as aminoethyl (meth)acrylates and N,N-dimethylaminoethyl (meth)acrylates; styrenic monomers such as styrene; olefinic monomers such as ethylene, propylene, isoprene, butadiene, and isobutylene; vinyl esters such as vinyl acetate and vinyl propionate; vinyl ethers such as methyl vinyl ether and ethyl vinyl ether; alkoxyalkyl (meth)acrylate monomers such as methoxyethyl (meth)acrylates and ethoxyethyl (meth)acrylates; alkylene glycol (meth)acrylate monomers such as poly(ethylene glycol) (meth)acrylates and polypropylene glycol (meth)acrylates; and polyfunctional monomers such as hexanediol di(meth)acrylates, (poly)ethylene glycol di(meth)acrylates, (poly)propylene glycol di(meth)acrylates, butyl di(meth)acrylates, and hexyl di(meth)acrylates.

(Compounds (B1) or Compounds (B2))

Compounds (B1) each having a hydrolyzable silicon-containing group and an ethylenically unsaturated bond-containing group (hereinafter also simply referred to as "compounds (B1)") and compounds (B2) each having a hydrolyzable silicon-containing group and a mercapto group (hereinafter also simply referred to as "compounds (B2)") are not specifically limited, as long as being compounds (e.g., monomer components) each having at least one hydrolyzable silicon-containing group per molecule and at least one ethylenically unsaturated bond-containing group or mercapto group per molecule. Each of different compounds (B1) and each of different compounds (B2) may be used alone or in combination, respectively.

In compounds (B1), the ethylenically unsaturated bond-containing group is a group containing an ethylenically unsaturated bond (ethylenic carbon-carbon double bond), and preferred examples thereof include vinyl group and isopropenyl group, of which vinyl group is more preferred.

Exemplary hydrolyzable silicon-containing groups in compounds (B1) and compounds (B2) include hydrolyzable

silyl groups. Exemplary hydrolyzable silyl groups include alkoxysilyl groups; hydrosilyl groups; and halogenated silyl groups such as chlorosilyl group, bromosilyl group, iodosilyl group, and fluorosilyl group. In hydrolyzable silyl groups, generally one to three, preferably two or three, groups or atoms (e.g., alkoxy groups, hydrogen atoms, and halogen atoms) are bonded to one silicon atom. Each of different groups or atoms (e.g., alkoxy groups and halogen atoms) may be bonded alone or in combination to the silicon atom.

Preferred hydrolyzable silyl groups for use herein are alkoxysilyl groups and hydrosilyl groups. As such hydrolyzable silyl groups, preferred are the hydrolyzable silyl groups (reactive silyl groups) represented by Formula (10) in WO 2005/054341 A1, such as hydrolyzable silyl groups represented by Formulae (10a) and (10b), of which hydrolyzable silyl groups represented by Formula (10b) are more preferred. Specific exemplary hydrolyzable silyl groups include those listed as examples of hydrolyzable silyl groups represented by Formula (10b) in WO 2005/054341 A1. Exemplary compounds (B1) and exemplary compounds (B2) therefore include, hydrolyzable silyl-containing compounds in which a hydrolyzable silyl group represented by Formula (10a) or (10b) is bonded with an ethylenically unsaturated bond-containing group or a mercapto group with or without the interposition of a bivalent organic group, as described in WO 2005/054341 A1.

Of hydrolyzable silyl groups for use herein, alkoxysilyl groups are preferred. Alkoxy moieties in alkoxysilyl groups are preferably alkoxy groups having one to four carbon atoms, such as methoxy group, ethoxy group, propoxy group, isopropoxy group, butoxy group, isobutyloxy group, s-butyloxy group, and t-butyloxy group. Among them, methoxy group, ethoxy group, propoxy group, and isopropoxy group are more preferred; methoxy group and ethoxy group are further preferred; and ethoxy group is particularly preferred.

Exemplary compounds (B1) and compounds (B2) usable herein include the vinyl-containing silane coupling agents and mercapto-containing silane coupling agents listed as specific examples in WO 2005/054341 A1. Specifically, exemplary compounds (B1) include vinyl-containing silane coupling agents listed in the description of silane compounds (D) described below as examples of silane compounds (D) represented by Formula (5b) wherein $R^{12}$ is vinyl group, a vinyl-alkyl group, or a (meth)acryloyloxyalkyl group. Exemplary compounds (B2) include mercapto-containing silane coupling agents listed in the description of silane compounds (D) described below as examples of silane compounds (D) represented by Formula (5b) wherein $R^{12}$ is a mercapto-alkyl group.

Compounds (B1) and compounds (B2) can be vinyl-containing silane coupling agents each having a monoalkoxysilyl group and mercapto-containing silane coupling agents each having a monoalkoxysilyl group, respectively; but they are preferably vinyl-containing silane coupling agents each having a dialkoxysilyl group or vinyl-containing silane coupling agents each having a trialkoxysilyl group, and mercapto-containing silane coupling agents each having a dialkoxysilyl group or mercapto-containing silane coupling agents each having a trialkoxysilyl group, respectively.

Of compounds (B1) and compounds (B2), compounds (B1) are preferably used herein.

(Urethane Polymers (C) Having Hydrolyzable Silicon-Containing Group)

Urethane polymers (C) having a hydrolyzable silicon-containing group (hereinafter also referred to as "urethane polymers (C)") are not particularly limited, as long as being polymers that have a urethane polymer chain containing a urethane bond (preferably as a backbone of the principle chain) per molecule and have at least one hydrolyzable silicon-containing group per molecule. Exemplary urethane polymers (C) for use herein include the urethane polymers (A) having a hydrolyzable silicon-containing group described in WO 2005/054341 A1. Each of different urethane polymers (C) may be used alone or in combination.

Urethane polymers (C) for use herein preferably have dispersibility or solubility in water and thereby preferably have one or more hydrophilic groups such as anionic groups, cationic groups, and nonionic groups. Of such hydrophilic groups, anionic groups are more preferred. Accordingly, preferred urethane polymers (C) are hydrolyzable sililated urethane polymers having at least one hydrophilic group per molecule (hydrophilic-group-containing hydrolyzable sililated urethane polymers).

Hydrolyzable silicon-containing groups in urethane polymers (C) are preferably hydrolyzable silyl groups. From this viewpoint, therefore, preferred urethane polymers (C) are urethane polymers having a hydrolyzable silyl group (hydrolyzable sililated urethane polymers). Exemplary hydrolyzable silyl groups in hydrolyzable sililated urethane polymers include alkoxysilyl groups; hydrosilyl groups; and halogenated silyl groups such as chlorosilyl group, bromosilyl group, iodosilyl group, and fluorosilyl group, as with the hydrolyzable silyl groups in the compounds (B1) and compounds (B2). In hydrolyzable silyl groups, generally one to three, preferably two or three, groups or atoms (e.g., alkoxy groups, hydrogen atoms, and halogen atoms) are bonded to one silicon atom. Each of different groups or atoms (e.g., alkoxy groups and halogen atoms) may be bonded alone or in combination to the silicon atom.

Hydrolyzable silyl groups in the hydrolyzable sililated urethane polymers are preferably alkoxysilyl group and hydrosilyl group, of which alkoxysilyl groups are more preferred. Exemplary alkoxy moieties in the alkoxysilyl groups include the alkoxy groups as listed in the compounds (B1) and compounds (B2), of which methoxy group, ethoxy group, propoxy group, and isopropoxy group are preferred, methoxy group and ethoxy group are more preferred, and ethoxy

group is particularly preferred.

Accordingly, of hydrolyzable sililated urethane polymers, alkoxysililated urethane polymers are preferred, of which hydrophilic-group-containing alkoxysililated urethane polymers (C1) are more preferred. Of the hydrophilic-group-containing alkoxysililated urethane polymers (C1), typically preferred are terminally alkoxysililated urethane polymer having a hydrophilic group which correspond to urethane polymers having a hydrophilic group, wherein at least part of terminal isocyanato groups is alkoxysililated.

More specific exemplary hydrophilic-group-containing alkoxysililated urethane polymers (C1) include a urethane polymer having a hydrophilic group and a terminal alkoxysilyl group, which is a product by reactions with following Components (C1-a), (C1-b), (C1-c), and (C1-d).

(C1-a): a compound having two or more active-hydrogen-containing groups but having no hydrophilic group;
(C2-b): a compound having a hydrophilic group and two or more active-hydrogen-containing groups;
(C1-c): a polyisocyanate compound; and
(C1-d): an alkoxysilane compound having an active-hydrogen-containing group.

(Compounds (C1-a) Having Two or More Active-Hydrogen-Containing Groups But Having No Hydrophilic Group)

Compounds (C1-a) having two or more active-hydrogen-containing groups but having no hydrophilic group (hereinafter also referred to as "active-hydrogen-containing compounds (C1-a)") are not particularly limited, as long as being compounds that have no hydrophilic group, such as an anionic group, a cationic group, or a nonionic group, per molecule and have at least two active-hydrogen-containing groups (groups each containing an active hydrogen) per molecule. Exemplary active-hydrogen-containing groups include hydroxyl group, primary amino group (unsubstituted amino group), secondary amino groups (mono-substituted amino groups), and mercapto group. A compound (C1-a) may contain one kind of active-hydrogen-containing groups or may contain two or more active-hydrogen-containing groups. Of active-hydrogen-containing groups herein, hydroxyl group, primary amino group, and secondary amino groups are preferred, of which hydroxyl group is more preferred. Accordingly, exemplary active-hydrogen-containing compounds (C1-a) include hydrophilic group-free polyol compounds, hydrophilic group-free polyamine compounds, and hydrophilic group-free polythiol compounds. Specific examples of such hydrophilic group-free polyol compounds, hydrophilic group-free polyamine compounds, and hydrophilic group-free polythiol compounds are those listed as examples of hydrophilic group-free polyol compounds, hydrophilic group-free polyamine compounds, and hydrophilic group-free polythiol compounds in the description of the isocyanate reactive compounds (A1-a) in WO 2005/054341 A1. Each of different active-hydrogen-containing compounds (C1-a) may be used alone or in combination.

Of active-hydrogen-containing compounds (C1-a) for use herein, preferred are hydrophilic group-free polyol compounds and hydrophilic group-free polyamine compounds, of which hydrophilic group-free polyol compounds (compounds each having no hydrophilic group but having two or more hydroxyl groups) are more preferred. Of hydrophilic group-free polyol compounds, preferred are polyetherpolyols and polyesterpolyols. Exemplary polyetherpolyols include poly(alkylene glycol)s such as poly(ethylene glycol)s, poly(propylene glycol)s, poly(tetramethylene glycol)s, and poly (hexamethylene glycol)s; and copolymers each containing two or more different alkylene oxides as monomer components (copolymers between an alkylene oxide and another alkylene oxide), such as ethylene oxide/propylene oxide copolymers. Of polyetherpolyols, those having a number-average molecular weight of from 500 to 30,000 are preferred, of which those having a number-average molecular weight of from 1,000 to 10,000 are more preferred.

Exemplary polyesterpolyols include polycondensates between a polyhydric alcohol and a polycarboxylic acid; ring-opened polymers of cyclic esters (lactones); and reaction products among three different components, i.e., a polyhydric alcohol, a polycarboxylic acid, and a cyclic ester. Of such polycondensates between a polyhydric alcohol and a polycarboxylic acid, exemplary polyhydric alcohols include ethylene glycol, diethylene glycol, propylene glycol, dipropylene glycol, trimethylene glycol, 1,4-tetramethylenediol, 1,3-tetramethylenediol, 2-methyl-1,3-trimethylenediol, 1,5-pentamethylenediol, neopentyl glycol, 1,6-hexamethylenediol, 3-methyl-1,5-pentamethylenediol, 2,4-diethyl-1,5-pentamethylenediol, glycerol, trimethylolpropane, trimethylolethane, cyclohexanediols (e.g., 1,4-cyclohexanediol), bisphenols (e.g., bisphenol-A), and sugar alcohols (e.g., xylitol and sorbitol). Exemplary polycarboxylic acids include maleic acid, adipic acid, sebacic acid, and phthalic acid. Exemplary cyclic esters include β-methyl-δ-valerolactone and ε-caprolactone. Exemplary polyesterpolyols further include castor oils having two or more active hydrogens. Of polyesterpolyols, those having a number-average molecular weight of from 500 to 25,000 are generally used.

(Compounds (C1-b) Having Hydrophilic Group and Two or More Active-Hydrogen-Containing Groups)

Compounds (C1-b) having a hydrophilic group and two or more active-hydrogen-containing groups (hereinafter also referred to as "active-hydrogen-containing compounds (C1-b)") are not particularly limited, as long as being compounds that have at least one hydrophilic group (an anionic group, a cationic group, or a nonionic group) per molecule

and at least two active-hydrogen-containing groups per molecule. In active-hydrogen-containing compounds (C1-b), exemplary hydrophilic groups include anionic groups such as carboxyl group and sulfo group; cationic groups including tertiary amino groups such as di-substituted amino groups; and nonionic groups including groups each containing a polyoxyalkylene chain such as a polyoxyethylene chain, a polyoxypropylene chain, or an oxyethylene/oxypropylene copolymer chain. Of hydrophilic groups, anionic groups are preferred, of which carboxyl group is more preferred. Exemplary active-hydrogen-containing groups include hydroxyl group, primary amino group, secondary amino groups, and mercapto group. A compound (C1-b) may contain one kind of active-hydrogen-containing groups or may contain two or more active-hydrogen-containing groups. Of active-hydrogen-containing groups for use herein, preferred are hydroxyl group, primary amino group, and secondary amino groups, of which hydroxyl group is more preferred. Accordingly, exemplary usable active-hydrogen-containing compounds (C1-b) include hydrophilic-group-containing polyol compounds, hydrophilic-group-containing polyamine compounds, and hydrophilic-group-containing polythiol compounds, of which hydrophilic-group-containing polyol compounds (compounds having a hydrophilic group and two or more hydroxyl groups) are more preferred. Each of different active-hydrogen-containing compounds (C1-b) may be used alone or in combination.

Thus, preferred as active-hydrogen-containing compounds (C1-b) are anionic-group-containing polyol compounds in which the hydrophilic group is an anionic group and the active-hydrogen-containing groups are hydroxyl groups (compounds having an anionic group and two or more hydroxyl groups). Of such anionic-group-containing polyol compounds, more preferred are polyhydroxycarboxylic acids represented by following Formula (1):

$$(HO)aL(COOH)b \qquad (1)$$

wherein L represents a hydrocarbon moiety having one to twelve carbon atoms; "a" denotes an integer of 2 or more; and "b" denotes an integer of 1 or more.

Exemplary polyhydroxycarboxylic acids include 2,2-dimethylolalkanoic acids such as 2,2-dimethylolpropionic acid, 2,2-dimethylolbutanoic acid, 2,2-dimethylolpentanoic acid, 2,2-dimethylolheptanoic acid, and 2,2-dimethyloloctanoic acid. Among them, 2,2-dimethylolpropionic acid and 2,2-dimethylolbutanoic acid are preferred as active-hydrogen-containing compounds (C1-b).

(Polyisocyanate Compounds (C1-c))

Polyisocyanate compounds (C1-c) (hereinafter referred to as "polyisocyanates (C1-c)") are not particularly limited, as long as being compounds having at least two isocyanato groups per molecule. Exemplary polyisocyanates (C1-c) include those listed as the polyisocyanate compounds (A1-c) in WO 2005/054341 A1, such as aliphatic polyisocyanates, alicyclic polyisocyanates, aromatic polyisocyanates, and aromatic-aliphatic polyisocyanates. Each of different polyisocyanates (C1-c) may be used alone or in combination.

Specifically, exemplary aliphatic polyisocyanates include 1,6-hexamethylene diisocyanate (HDI). Exemplary alicyclic polyisocyanates include 4,4'-methylenebis(cyclohexyl isocyanate) (H-MDI), isophorone diisocyanate (IPDI), and norbornene diisocyanate (NBDI). Exemplary aromatic-aliphatic polyisocyanates include 1,3-xylylene diisocyanate and 1,4-xylylene diisocyanate. Exemplary aromatic polyisocyanates include 4,4'-diphenylmethane diisocyanate (MDI) and 2,4- or 2,6-tolylene diisocyanates (TDI). One or more diisothiocyanate compounds such as phenyl diisothiocyanate may be used in combination with polyisocyanates (C1-c).

(Alkoxysilane Compounds (C1-d) Having Active-Hydrogen-Containing Group)

Alkoxysilane compounds (C1-d) having an active-hydrogen-containing group (hereinafter also referred to as "active-hydrogen-containing alkoxysilanes (C1-d)") are not particularly limited, as long as being silane compounds each having at least one active-hydrogen-containing group per molecule and at least one alkoxy group per molecule. Each of different active-hydrogen-containing alkoxysilanes (C1-d) may be used alone or in combination.

In active-hydrogen-containing alkoxysilanes (C1-d), exemplary active-hydrogen-containing groups include primary amino group, secondary amino groups, mercapto group, isocyanato group, and hydroxyl group, of which amino groups such as primary amino group and secondary amino groups, and mercapto group are preferred. An active-hydrogen-containing alkoxysilane (C1-d) may contain one or more active-hydrogen-containing groups of one kind or may contain active-hydrogen-containing groups of two or more different kinds. Accordingly, exemplary preferred active-hydrogen-containing alkoxysilanes (C1-d) include amino-containing alkoxysilane compounds (C1-d1) each having an amino group (primary amino group or secondary amino group) as an active-hydrogen-containing group (hereinafter also referred to as "amino-containing alkoxysilanes (C1-d1)"); and mercapto-containing alkoxysilane compounds (C1-d2) each having a mercapto group as an active-hydrogen-containing group (hereinafter also referred to as "mercapto-containing alkoxysilanes (C1-d2)").

Exemplary active-hydrogen-containing alkoxysilanes (C1-d) include those as with the isocyanate-reactive-group-containing alkoxysilane compounds (A1-d) listed in WO 2005/054341 A1. More specifically, exemplary amino-containing alkoxysilane (C1-d1) include, of the isocyanate-reactive-group-containing alkoxysilane compounds (A1-d) in WO 2005/054341 A1, those listed as examples of amino-containing alkoxysilanes represented by Formula (6a) having a primary amino group alone as an isocyanate-reactive group; those listed as examples of amino-containing alkoxysilanes represented by Formula (6b) having a primary amino group and a secondary amino group as isocyanate-reactive groups; and those listed as examples of amino-containing alkoxysilanes represented by Formula (6c) having a secondary amino group alone as an isocyanate-reactive group. Exemplary mercapto-containing alkoxysilanes (C1-d2) include, of the isocyanate-reactive-group-containing alkoxysilane compounds (A1-d) in WO 2005/054341 A1, those listed as examples of mercapto-containing alkoxysilanes represented by Formula (6d) having a mercapto group as an isocyanate-reactive group.

Of active-hydrogen-containing alkoxysilanes (C1-d) for use herein, amino group-containing alkoxysilanes (C1-d1) are preferred, of which more preferred are alkoxysilane compounds having at least a secondary amino group as an active-hydrogen-containing group (hereinafter also referred to as "ester-modified amino-containing alkoxysilanes (C1-d4)"), which ester-modified amino-containing alkoxysilanes (C1-d4) are reaction products of an alkoxysilane compound having at least a primary amino group as an active-hydrogen-containing group (hereinafter also referred to as "primary amino-containing alkoxysilane") with an unsaturated carboxylic acid ester (C1-d3). The primary amino-containing alkoxysilane more preferably contains both a primary amino group and a secondary amino group as active-hydrogen-containing groups. Of ester-modified amino-containing alkoxysilanes (C1-d4), preferred are secondary amino-containing alkoxysilane compounds as reaction products of an alkoxysilane compound having at least a primary amino group with an unsaturated carboxylic acid ester (C1-d3). Exemplary alkoxysilane compounds having at least a primary amino group include alkoxysilane compounds each having a primary amino group alone; and alkoxysilane compounds each having both a primary amino group and a secondary amino group. Accordingly, exemplary preferred ester-modified amino-containing alkoxysilanes (C1-d4) include secondary amino-containing alkoxysilane compounds as reaction products of an alkoxysilane compound having a primary amino group alone with an unsaturated carboxylic acid ester (C1-d3); and secondary amino-containing alkoxysilane compounds as reaction products of an alkoxysilane compound having both a primary amino group and a secondary amino group with an unsaturated carboxylic acid ester (C1-d3), of which the latter secondary amino-containing alkoxysilane compounds are more preferred.

Of ester-modified amino-containing alkoxysilanes (C1-d4), exemplary unsaturated carboxylic acid esters (C1-d3) include compounds listed as examples of unsaturated carboxylic acid esters (A1-d3) in description relating to the ester-modified amino-containing alkoxysilanes (A1-d4) as the isocyanate-reactive-group-containing alkoxysilane compounds (A1-d) in WO 2005/054341 A1. Among them, acrylic esters and maleic diesters are preferred. Each of different unsaturated carboxylic acid esters (C1-d3) may be used alone or in combination.

Thus, exemplary preferred active-hydrogen-containing alkoxysilanes (C1-d) include secondary amino-containing alkoxysilane compounds respectively represented by following Formulae (2a), (2b, and (2c):

[Chemical Formula 1]

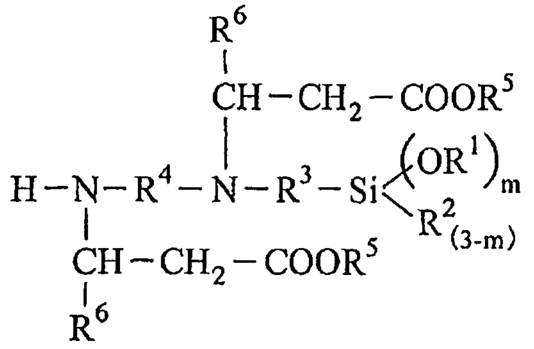

(2a)

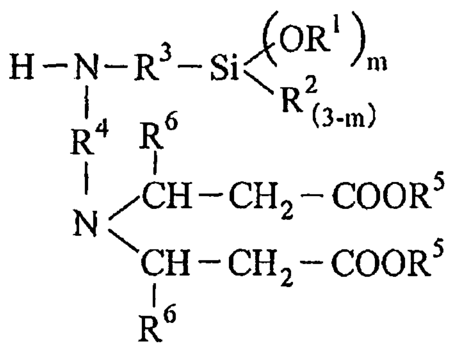

(2b)

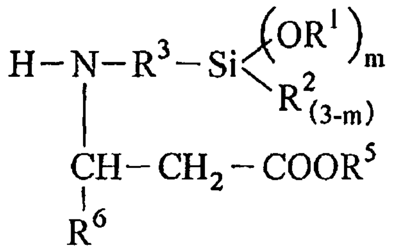

(2c)

wherein R1 is an alkyl group having one to six carbon atoms; R2 is a hydrogen atom or an alkyl group having one to six carbon atoms; R3 and R4 may be the same as or different from each other and are each an alkylene group having one to ten carbon atoms or an arylene group; R5 is an alkyl group having one to twenty carbon atoms, a cycloalkyl group, an aryl group, or an aralkyl group; R6 is a hydrogen atom or a group represented by "-COOR5", where R5 is as defined above; and "m" is an integer of from 1 to 3.

In Formulae (2a) to (2c), exemplary alkyl groups having one to six carbon atoms as R1 and exemplary alkyl groups having one to six carbon atoms as R2 include methyl group, ethyl group, propyl group, isopropyl group, butyl group, isobutyl group, and t-butyl group. Exemplary alkylene groups having one to ten carbon atoms as R3 and R4 include methylene group, ethylene group, and trimethylene group. Exemplary arylene groups as R3 and R4 include phenylene group. Exemplary alkyl groups having one to twenty carbon atoms as R5 include methyl group, ethyl group, propyl group, butyl group, isobutyl group, t-butyl group, hexyl group, octyl group, 2-ethylhexyl group, isooctyl group, decyl group, isodecyl group, dodecyl group, tetradecyl group, hexadecyl group, and octadecyl group. Exemplary cycloalkyl groups as R5 include cyclohexyl group. Exemplary aryl groups as R5 include phenyl group. Exemplary aralkyl groups as R5 include benzyl group. The group as R6 represented by "-COOR5" is an alkoxy-carbonyl group whose alkoxy moiety has one to twenty carbon atoms, a cycloalkyloxycarbonyl group, an aryloxycarbonyl group, or an aralkyloxycarbonyl group, and R5 in these groups is as defined above.

These ester-modified amino-containing alkoxysilanes (C1-d4) are compounds each prepared via a Michael addition reaction of the nitrogen atom of amino group of a primary amino-containing alkoxysilane to the unsaturated bond (carbon-carbon double bond) of an unsaturated carboxylic acid ester (C1-d3). The reaction may be carried out in the presence of, or in the absence of, a solvent. The reaction may be conducted upon the application of heat and/or a pressure (load).

Of active-hydrogen-containing alkoxysilanes (C1-d) for use herein, alkoxysilane compounds having at least a secondary amino group as an active-hydrogen-containing group are preferred, of which ester-modified amino-containing

alkoxysilanes (C1-d4) represented by Formulae (2a) to (2c) are more preferred.

(Hydrophilic-Group-Containing Alkoxysililated Urethane Polymers (C1))

Accordingly, preferred as hydrophilic-group-containing alkoxysililated urethane polymers (C1) are urethane polymers having an anionic group and an alkoxysilyl group, represented by following Formula (3);

[Chemical Formula 2] $A(NH\text{-}CO\text{-}X)_n$ (3)

wherein "A" is a residue corresponding to a backbone of a urethane polymer containing an anionic group; the nitrogen atom bonded with "A" is a nitrogen atom derived from a terminal isocyanato group of the urethane polymer containing an anionic group; X is a group having an alkoxysilyl group, represented by any one of following Formulae (4a), (4b), and (4c); and "n" is an integer of from 1 to 20:

[Chemical Formula 3]

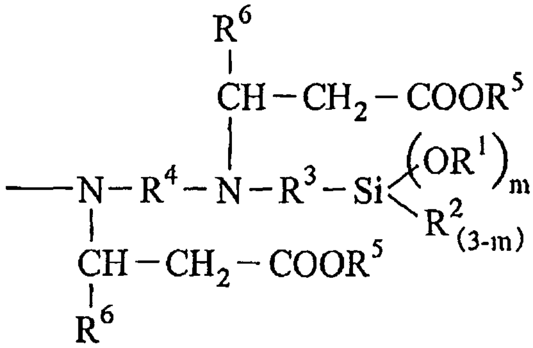

(4a)

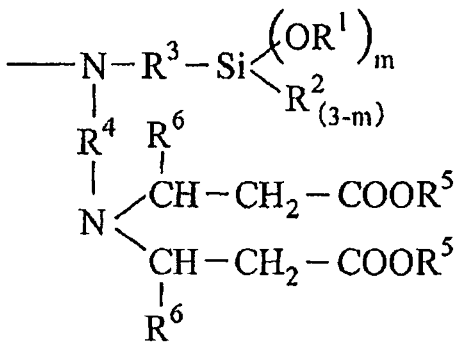

(4b)

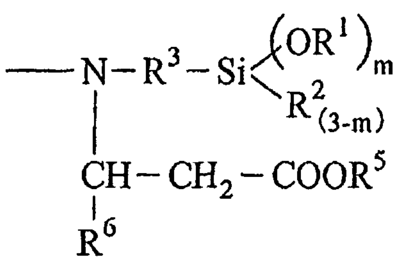

(4c)

wherein R1 to R6, and "m" are as defined above.

Exemplary hydrophilic-group-containing alkoxysililated urethane polymers (C1) include terminally alkoxysililated hydrophilic-group-containing urethane prepolymers each as a reaction product of a hydrophilic-group-containing urethane prepolymer with an active-hydrogen-containing alkoxysilane (C1-d), with at least part of terminal isocyanato

groups of the hydrophilic-group-containing urethane prepolymer being alkoxysililated. The hydrophilic-group-containing urethane prepolymer herein is a reaction product among an active-hydrogen-containing compound (C1-a), an active-hydrogen-containing compound (C1-b), and a polyisocyanate (C1-c).

Processes for producing hydrophilic-group-containing alkoxysililated urethane polymers (C1) have been known and include, for example, the production methods specifically described as relating to the hydrophilic-group-containing alkoxysililated urethane polymers (A1) in WO 2005/054341 A1.

The proportions of the respective components, i.e., active-hydrogen-containing compounds (C1-a), active-hydrogen-containing compounds (C1-b), polyisocyanates (C1-c), and active-hydrogen-containing alkoxysilanes (C1-d) in hydrophilic-group-containing alkoxysililated urethane polymers (C1) are not particularly limited and may be, for example, the proportions as specified relating to the hydrophilic-group-containing alkoxysililated urethane polymers (A1) in WO 2005/054341 A1.

Specifically, for example, the ratio of polyisocyanates (C1-c) to the total of active-hydrogen-containing compounds (C1-a) and active-hydrogen-containing compounds (C1-b) may be selected within such a range that the ratio (equivalent ratio) of isocyanato groups in the polyisocyanates (C1-c) to the total of active-hydrogen-containing groups in the active-hydrogen-containing compounds (C1-a) and active-hydrogen-containing compounds (C1-b) (NCO/NCO-reactive group) is more than 1 and 2.0 or less, preferably from 1.02 to 1.5, and more preferably from 1.05 to 1.4. Alternatively, the content of polyisocyanates (C1-c) may be such that the isocyanate content of the hydrophilic-group-containing urethane prepolymer is from 0.3 to 7.0 percent by mass, preferably from 0.4 to 4.0 percent by mass, and more preferably from 0.5 to 3.0 percent by mass.

An alkoxysililated urethane polymer (C1) having a hydrophilic group, if having an anionic group as the hydrophilic group [namely, in the case of an anionic-group-containing alkoxysililated urethane polymer], preferably has such a content of active-hydrogen-containing compounds (C1-b) as to have an anionic group content of 0.4 meq/g or more, for example, from 0.4 to 0.7 meq/g, and preferably from 0.4 to 0.6 meq/g. An anionic-group-containing alkoxysililated urethane polymer, if having an excessively high anionic group content, may cause insufficient water resistance. In contrast, an anionic-group-containing alkoxysililated urethane polymer, if having an excessively low anionic group content (e.g., less than 0.4 meq/g), may not help to provide sufficient stability in dispersion.

Hydrophilic-group-containing alkoxysililated urethane polymers (C1) preferably contain active-hydrogen-containing alkoxysilanes (C1-d) in such a content that the silicon content of the urethane polymers (C1) is, for example, from 0.02 to 10 percent by mass, preferably from 0.03 to 3 percent by mass, and more preferably from 0.05 to 2 percent by mass. An alkoxysililated urethane polymer (C1) having a hydrophilic group, if having an excessively high silicon content (e.g., more than 10 percent by mass), may cause insufficient stability of the resulting composition. In contrast, an alkoxysililated urethane polymer (C1) having a hydrophilic group, if having an excessively low silicon content (e.g., less than 0.02 percent by mass), may impede efficient formation of an alkoxysililated urethane polymer (C1) having a hydrophilic group and may fail to provide desired advantages.

In a preferred embodiment, hydrophilic-group-containing alkoxysililated urethane polymers (C1) represented by Formula (3) each have a silicon content in the alxoxysilyl-containing group as X of from 0.1 to 1.5 percent by mass. An alkoxysililated urethane polymer (C1) having a hydrophilic group represented by Formula (3), if having a silicon content in the alxoxysilyl-containing group as X of less than 0.1 percent by mass, may cause insufficient natural finishing capability to set the hair in a natural finish. In contrast, an alkoxysililated urethane polymer (C1) having a hydrophilic group represented by Formula (3), if having a silicon content in the alxoxysilyl-containing group as X of more than 1.5 percent by mass, may cause the set hair to have a coarse/stiff finish.

(Silane Compounds (D))

Silane compounds (D) having a hydrolyzable silicon-containing group (hereinafter also referred to as "silane compounds (D)") are not particularly limited, as long as being silane compounds that have at least one hydrolyzable silicon-containing group per molecule. Each of different silane compounds (D) may be used alone or in combination.

In silane compounds (D), exemplary hydrolyzable silicon-containing groups include hydrolyzable silyl groups including alkoxysilyl groups; hydrosilyl groups; and halogenated silyl groups such as chlorosilyl group, bromosilyl group, iodosilyl group, and fluorosilyl group, as in the compounds (B1) and the compounds (B2). In such hydrolyzable silyl groups, generally one to three, preferably two or three, groups or atoms (e.g., alkoxy groups, hydrogen atoms, and halogen atoms) are bonded to one silicon atom. Each of different groups or atoms (e.g., alkoxy groups and halogen atoms) may be bonded alone or in combination to the silicon atom. Specifically, one group (typified by an alkoxy group) or atom may be bonded to one silicon atom, or two or more different groups or atoms may be bonded to one silicone atom.

Of hydrolyzable silyl groups for use herein, alkoxysilyl groups and hydrosilyl groups are preferred, of which alkoxysilyl groups are more preferred. As such hydrolyzable silyl groups, the hydrolyzable silyl groups (reactive silyl groups) represented by Formula (11) in WO 2005/054341 A1 are preferred, of which the hydrolyzable silyl groups represented by Formulae (11a) to (11c) are more preferred.

Of silane compounds (D), preferred are the silane compounds (D) represented by Formula (12a) and the silane compounds (D) represented by Formula (12b) in WO 2005/054341 A1. Specifically, of silane compounds (D), preferred are silane compounds (D) represented by following Formula (5a) and silane compounds (D) represented by following Formula (5b):

[Chemical Formula 4]

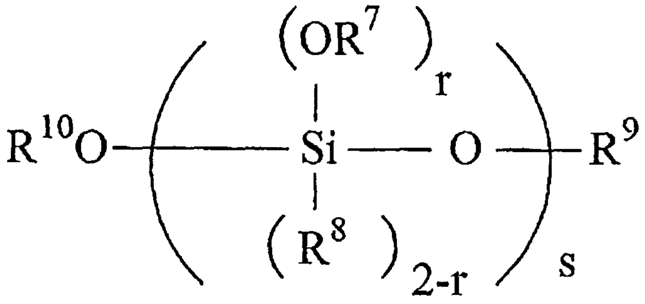

(5a)

wherein R7 is a hydrogen atom or a hydrocarbon group; R8 is a hydrogen atom or a hydrocarbon group; R9 and R10 are the same as or different from each other and are each a hydrogen atom or a hydrocarbon group; "r" is 1 or 2; and "s" is an integer of 1 or more,

[Chemical Formula 5]

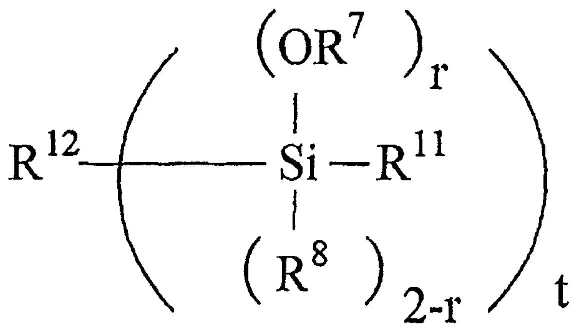

(5b)

wherein R11 is an OR7 or R8; R12 is an organic group; "t" is an integer of 1 or more; and R7, R8, and "r" are as defined above.

In Formula (5a), exemplary hydrocarbon groups as R7 include aliphatic hydrocarbon groups, alicyclic hydrocarbon groups, and aromatic hydrocarbon groups, of which aliphatic hydrocarbon groups are preferred, and alkyl groups are more preferred. Specifically, exemplary alkyl groups as R7 include alkyl groups having about one to about twenty carbon atoms, such as methyl group, ethyl group, propyl group, isopropyl group, butyl group, isobutyl group, t-butyl group, s-butyl group, n-pentyl group, hexyl group, heptyl group, octyl group, 2-ethylhexyl group, nonyl group, decyl group, undecyl group, dodecyl group, tridecyl group, tetradecyl group, pentadecyl group, hexadecyl group, heptadecyl group, and octadecyl group. Among them, alkyl groups having one to ten carbon atoms are preferred, those having one to six carbon atoms are more preferred, and those having one to four carbon atoms are further preferred.

Exemplary hydrocarbon groups as R8 include aliphatic hydrocarbon groups, alicyclic hydrocarbon groups, and aromatic hydrocarbon groups, of which aliphatic hydrocarbon groups are preferred, and alkyl groups are more preferred. Exemplary alkyl groups as R8 include the alkyl groups exemplified as alkyl groups as R7, of which alkyl groups having one to ten carbon atoms are preferred, those having one to six carbon atoms are more preferred, and those having one to four carbon atoms are further preferred.

Hydrocarbon groups as R7 and R8 may each have one or more substituents. The hydrocarbon groups R7 and R8 may each be combined with another hydrocarbon group (e.g., a hydrocarbon group as R7 or R8 bonded to another silicon atom) typically through the substituent to form a ring, such as an aromatic ring or a non-aromatic ring. Each of the groups R7 and R8 may be bonded to another R7 or R8 which is bonded to the same or different silicon atom.

Of hydrocarbon groups as R9 and R10, aliphatic hydrocarbon groups are preferred, of which alkyl groups the same as or different from R9 and R10 are more preferred. Each of R9 and R10 may be a hydrocarbon group the same as or different from R7.

The repetition number "r" is 1 or 2 and is preferably 2. When "r" is 2, it means that there is no R8, and two "-OR7" groups are bonded to the silicon atom in Formula (5a). When "s" is 1, a silane compound (D) represented by Formula (5a) is a monomer; and when "s" is an integer of 2 or more, a silane compound (D) represented by Formula (5a) is a multimer such as an oligomer or a polymer.

Exemplary silane compounds (D) represented by Formula (5a) include those listed as examples of the silane compounds represented by Formula (12a) in WO 2005/054341 A1.

Among silane compounds (D) represented by Formula (5a), exemplary silane compounds (D) in the form of monomers include tetraalkoxysilanes such as tetramethoxysilane, tetraethoxysilane, tetrapropoxysilane, tetraisopropoxysilane, and tetrabutoxysilane; alkoxytrialkoxysilanes such as methoxytriethoxysilane; and dialkoxydialkoxysilanes such as dimethoxydiethoxysilane. Exemplary silane compounds (D) in the form of multimers include poly(tetraalkoxysilane)s such as poly(tetramethoxysilane)s, poly(tetraethoxysilane)s, poly(tetrapropoxysilane)s, poly(tetraisopropoxysilane)s, and poly(tetrabutoxysilane)s; poly(alkoxyalkoxysilane)s such as poly(methoxyethoxysilane)s; poly(alkoxysilane) s such as poly(methoxysilane)s, poly(ethoxysilane)s, poly(propoxysilane)s, poly(isopropoxysilane)s, and poly(butoxysilane)s; and poly(alkoxyalkylsilane)s such as poly(methoxymethylsilane)s, poly(methoxyethylsilane)s, and poly (ethoxymethylsilane)s.

In Formula (5b), R11 is an OR7 or R8. Plural OR7s and plural R8s, if bonded to one silicon atom, may be the same as or different from each other, respectively.

The organic group as R12 corresponds to R30 in Formula (12b) in WO 2005/054341 A1 and may be suitably selected from among the groups exemplified as R30. Specifically, exemplary organic groups as R12 include hydrocarbon groups; and hetero atom-containing groups each having an atom other than carbon atom (oxygen atom, nitrogen atom, and/or sulfur atom) in the principal chain of the hydrocarbon groups. These hydrocarbon groups and hetero atom-containing groups may be monovalent or multivalent. Exemplary preferred organic groups as R12 are vinyl group and mercapto group; as well as vinyl-alkyl groups, vinyl-(alkyl)-aryl groups, vinyl-(alkyl)-cycloalkyl groups, (meth)acryloyl groups, (meth)acryloyloxyalkyl groups (vinyl-carbonyloxyalkyl groups), (meth)acryloyloxyaryl groups, mercapto-alkyl groups, mercapto-(alkyl)-aryl groups, and mercapto-(alkyl)-cycloalkyl groups.

The repetition number "t" is not particularly limited, as long as being an integer of 1 or more, but is preferably an integer of from 1 to 4, more preferably from 1 or 2, and particularly preferably 1. When "t" is an integer of 2 or more, it means that two or more hydrolyzable silicon-containing groups are bonded to the organic group as R12.

Exemplary silane compounds (D) represented by Formula (5b) include those exemplified as the silane compounds represented by Formula (12b) in WO 2005/054341 A1.

Specifically, exemplary silane compounds (D) represented by Formula (5b), in which R12 is an alkyl group, include alkyltrialkoxysilanes such as methyltrimethoxysilane, ethyltrimethoxysilane, methyltriethoxysilane, methyltriethoxysilane, methyltripropoxysilane, ethyltripropoxysilane, propyltripropoxysilane, isopropyltripropoxysilane, butyltripropoxysilane, methyltriisopropoxysilane, ethyltriisopropoxysilane, propyltriisopropoxysilane, methyltributoxysilane, ethyltributoxysilane, and propyltributoxysilane; dialkyldialkoxysilanes such as dimethyldimethoxysilane, dimethyldiethoxysilane, dimethyldipropoxysilane, dimethyldiisopropoxysilane, dimethyldibutoxysilane, diethyldimethoxysilane, diethyldiethoxysilane, diethyldipropoxysilane, diethyldiisopropoxysilane, diethyldibutoxysilane, dipropyldimethoxysilane, dipropyldiethoxysilane, and dipropyldipropoxysilane; and trialkylalkoxysilanes corresponding to these.

When R12 is an alkyl group having one or more substituents such as glycidoxy group, isocyanato group, and amino group, exemplary silane compounds (D) represented by Formula (5b) include those corresponding to the above examples as silane compounds (D) represented by Formula (5b), in which R12 is an alkyl group.

Exemplary silane compounds (D) represented by Formula (5b), in which R12 is a vinyl group, include vinyltrialkoxysilanes such as vinyltrimethoxysilane, vinyltriethoxysilane, vinyltripropoxysilane, vinyltriisopropoxysilane, and vinyltributoxysilane; (vinyl)alkyldialkoxysilanes such as vinylmethyldimethoxysilane, vinylmethyldiethoxysilane, vinylmethyldipropoxysilane, vinylmethyldiisopropoxysilane, vinylmethyldibutoxysilane, vinylethyldimethoxysilane, vinylethyldiethoxysilane, vinylethyldipropoxysilane, vinylethyldiisopropoxysilane, vinylethyldibutoxysilane, vinylpropyldimethoxysilane, vinylpropyldiethoxysilane, vinylpropyldipropoxysilane, vinylpropyldiisopropoxysilane, and vinylpropyldibutoxysilane; and (vinyl)dialkyl(mono)alkoxysilanes corresponding to these.

Exemplary silane compounds (D) represented by Formula (5b), in which R12 is a vinyl-alkyl group, include vinylalkyltrialkoxysilanes such as vinylmethyltrimethoxysilane, vinylmethyltriethoxysilane, β-vinylethyltrimethoxysilane, β-vinylethyltriethoxysilane, β-vinylethyltripropoxysilane, β-vinylethyltriisopropoxysilane, β-vinylethyltributoxysilane, γ-vinylpropyltrimethoxysilane, γ-vinylpropyltriethoxysilane, γ-vinylpropyltripropoxysilane, γ-vinylpropyltriisopropoxysilane, and γ-vinylpropyltributoxysilane; (vinylalkyl)alkyldialkoxysilanes such as β-vinylethylmethyldimethoxysilane, β-vinylethylmethyldiethoxysilane, γ-vinylpropylmethyldimethoxysilane, γ-vinylpropylmethyldiethoxysilane, γ-vinylpropylmethyldipropoxysilane, γ-vinylpropylmethyldiisopropoxysilane, γ-vinylpropylmethyldibutoxysilane, γ-vinylpropylethyldimethoxysilane, γ-vinylpropylethyldiethoxysilane, γ-vinylpropylethyldipropoxysilane, γ-vinylpropylethyldiisopropoxysilane, and γ-vinylpropylethyldibutoxysilane; and (vinylalkyl)dialkyl(mono)alkoxysilanes corresponding to these.

Exemplary silane compounds (D) represented by Formula (5b), in which R12 is a (meth)acryloyloxyalkyl group, include (meth)acryloxyalkyl-trialkoxysilanes such as (meth)acryloxymethyl-trimethoxysilanes, (meth)acryloxymethyl-triethoxysilanes, 2-(meth)acryloxyethyl-trimethoxysilanes, 2-(meth)acryloxyethyl-triethoxysilanes, 2-(meth)acryloxyethyltripropoxysilanes, 2-(meth)acryloxyethyl-triisopropoxysisilanes, 2-(meth)acryloxyethyl-tributoxysisilanes, 3-(meth)acryloxypropyl-trimethoxysilanes, 3-(meth)acryloxypropyl-triethoxysilanes, 3-(meth)acryloxypropyl-tripropoxysilanes, 3-(meth)acryloxypropyl-triisopropoxysilanes, and 3-(meth)acryloxypropyl-tributoxysilanes; (meth)acryloxyalkyl-alkyldialkoxysilanes such as (meth)acryloxymethyl-methyldimethoxysilanes, (meth)acryloxymethyl-methyldiethoxysilanes, 2-(meth)acryloxyethyl-methyldimethoxysilanes, 2-(meth)acryloxyethyl-methyldiethoxysilanes, 2-(meth)acryloxyethyl-methyldipropoxysilanes, 2-(meth)acryloxyethyl-methyldiisopropoxysilanes, 2-(meth)acryloxyethyl-methyldibutoxysilanes, 3-(meth)acryloxypropyl-methyldimethoxysilanes, 3-(meth)acryloxypropyl-methyldiethoxysilanes, 3-(meth)acryloxypropyl-methyldipropoxysilanes, 3-(meth)acryloxypropyl-methyldiisopropoxysilanes, 3-(meth)acryloxypropyl-methyldibutoxysilanes, 3-(meth)acryloxypropyl-ethyldimethoxysilanes, 3-(meth)acryloxypropyl-ethyldiethoxysilanes, 3-(meth) acryloxypropyl-ethyldipropoxysilanes, 3-(meth)acryloxypropyl-ethyldiisopropoxysilanes, 3-(meth)acryloxypropyl-ethyldibutoxysilanes, 3-(meth)acryloxypropyl-propyldimethoxysilanes, 3-(meth)acryloxypropyl-propyldiethoxysilanes, 3-(meth)acryloxypropyl-propyldipropoxysilanes, 3-(meth)acryloxypropyl-propyldiisopropoxysilanes, and 3-(meth)acryloxypropyl-propyldibutoxysilanes; and (meth)acryloxyalkyl-dialkyl(mono)alkoxysilanes corresponding to these.

Exemplary silane compounds (D) represented by Formula (5b), in which R12 is a mercapto-alkyl group, include mercaptoalkyltrialkoxysilanes such as mercaptomethyltrimethoxysilane, mercaptomethyltriethoxysilane, β-mercaptoethyltrimethoxysilane, β-mercaptoethyltriethoxysilane, β-mercaptoethyltripropoxysilane, β-mercaptoethyltriisopropoxysilane, β-mercaptoethyltributoxysilane, γ-mercaptopropyltrimethoxysilane, γ-mercaptopropyltriethoxysilane, γ-mercaptopropyltripropoxysilane, γ-mercaptopropyltriisopropoxysilane, and γ-mercaptopropyltributoxysilane; (mercaptoalkyl) alkyldialkoxysilanes such as β-mercaptoethylmethyldimethoxysilane, β-mercaptoethylmethyldiethoxysilane, γ-mercaptopropylmethyldimethoxysilane, γ-mercaptopropylmethyldiethoxysilane, γ-mercaptopropylmethyldipropoxysilane, γ-mercaptopropylmethyldiisopropoxysilane, γ-mercaptopropylmethyldibutoxysilane, γ-mercaptopropylethyldimethoxysilane, γ-mercaptopropylethyldiethoxysilane, γ-mercaptopropylethyldipropoxysilane, γ-mercaptopropylethyldiisopropoxysilane, and γ-mercaptopropylethyldibutoxysilane; and (mercaptoalkyl)dialkyl(mono)alkoxysilanes corresponding to these.

Exemplary silane compounds (D) represented by Formulae (5a) and (5b) further include hydroxyl-containing silane compounds that correspond to the above-exemplified alkoxy-containing silane compounds, with alkoxy groups converted into hydroxyl groups.

Of such silane compounds (D), silane compounds (D) represented by Formula (5b) act as vinyl-containing silane coupling agents when the organic group as R12 is a vinyl-containing group such as vinyl group, a vinyl-alkyl group, a vinyl-(alkyl)-aryl group, a vinyl-(alkyl)-cycloalkyl group, a (meth)acryloyl group, a (meth)acryloyloxyalkyl group, or a (meth)acryloyloxyaryl group. Silane compounds (D) represented by Formula (5b) act as mercapto-containing silane coupling agents when the organic group as R12 is a mercapto-containing group such as mercapto group, a mercapto-alkyl group, a mercapto-(alkyl)-aryl group, or a mercapto-(alkyl)-cycloalkyl group.

When one silane compound (D) is included both in silane compounds (D) represented by Formula (5a) and in silane compounds (D) represented by Formula (5b), the silane compound (D) may be suitably categorized into a silane compound (D) represented by either Formula (5a) or Formula (5b).

When silane compounds (D) represented by Formula (5b) are vinyl-containing silane coupling agents and mercapto-containing silane coupling agents, the vinyl-containing silane coupling agents and mercapto-containing silane coupling agents can also be used as compounds (B1) and compounds (B2), respectively. In other words, silane compounds (D) include compounds (B1) and compounds (B2). Thus, vinyl-containing silane coupling agents and mercapto-containing silane coupling agents may be used herein not as compounds (B1) and/or compounds (B2) but as silane compounds (D); may be used not as silane compounds (D) but as compounds (B1) and/or compounds (B2); or may be used both as silane compounds (D) and as compounds (B1) and/or compounds (B2). When such silane coupling agents and mercapto-containing silane coupling agents are used at least as silane compounds (D), it is important that the silane coupling agents and mercapto-containing silane coupling agents have such a structure in which two or more (two or three) hydrolyzable groups, such as alkoxy groups, are bonded to one silicon atom, as typically in vinyl-(alkyl)-trialkoxysilanes, vinyl-(alkyl)-alkyldialkoxysilanes, mercaptoalkyl-trialkoxysilanes, and mercaptoalkyl-alkyldialkoxysilanes.

Of silane compounds (D) for use herein, preferred are alkoxy-containing silane compounds (D1). As such silane compounds (D), vinyl-containing silane coupling agents as with compounds (B1) and mercapto-containing silane coupling agents as with compounds (B2) are preferred, of which vinyl-containing silane coupling agents as with compounds (B1) are more preferred.

When an importance is placed on the natural feeling and smooth touch, a silane compound (D) for use herein preferably contains a dialkoxysilyl group-containing silane compound (D1-1). Use of a dialkoxysilyl-containing silane

compound (D1-1) as the silane compound (D) helps the hair cosmetic to exhibit further improved touch, such as less coarse/stiff feeling and less sticky feeling, of the set hair, and this enables the hair to be set in a further natural finish.

When an importance is placed on the hair styling capability and the hair style retentivity, a silane compound (D) for use herein preferably contains a trialkoxysilyl-containing silane compound (D1-2). Use of a trialkoxysilyl-containing silane compound (D1-2) as the silane compound (D) helps the hair cosmetic to exhibit furthermore superior hair styling capability for setting the hair to a predetermined hair style and to exhibit further more superior hair style retentivity for maintaining the set hair style even under severe conditions of high humidity.

The silane compound (D) for use herein may contain both a dialkoxysilyl-containing silane compound (D1-1) and a trialkoxysilyl-containing silane compound (D1-2). Combination use of a dialkoxysilyl-containing silane compound (D1-1) and a trialkoxysilyl-containing silane compound (D1-2) as the silane compound (D) helps the hair cosmetic to exhibit further higher hair style retentivity and to realize further more pliable and smooth touch of the hair in a set hair style. Additionally, the combination use helps the hair cosmetic to show no or substantially no sticky feeling during a duration from the application of the hair cosmetic to drying. The resulting hair cosmetic thereby shows further less sticky feeling during a duration from the application of the hair cosmetic to drying.

Of such dialkoxysilyl-containing silane compounds (D1-1), preferred are dimethyldimethoxysilane, dimethyldiethoxysilane, dimethyldipropoxysilane, diethyldimethoxysilane, diethyldiethoxysilane, diethyldipropoxysilane, 3-chloropropyldimethoxymethylsilane, 3-chloropropyldiethoxymethylsilane, diethoxymethylvinylsilane, diethoxydivinylsilane, γ-methacryloxypropylmethyldimethoxysilane, and γ-methacryloxypropylmethyldiethoxysilane.

Of trialkoxysilyl-containing silane compounds (D1-2), preferred are methyltrimethoxysilane, ethyltrimethoxysilane, methyltriethoxysilane, methyltriethoxysilane, methyltripropoxysilane, ethyltripropoxysilane, 3-chloropropyltrimethoxysilane, 3-chloropropyltriethoxysilane, trimethoxyvinylsilane, triethoxyvinylsilane, γ-methacryloxypropyltrimethoxysilane, and γ-methacryloxypropyltriethoxysilane.

When the silane compound (D) contains both a dialkoxysilyl-containing silane compound (D1-1) and a trialkoxysilyl-containing silane compound (D1-2), the ratio of the dialkoxysilyl-containing silane compound (D1-1) to the trialkoxysilyl-containing silane compound (D1-2) may be selected within a range of from 0.1/99.9 to 100/0, and preferably from 15/85 to 85/15. A dialkoxysilyl-containing silane compound (D1-1), if contained in an excessively small ratio to a trialkoxysilyl-containing silane compound (D1-2), may cause an insufficient natural finishing capability. In contrast, a trialkoxysilyl-containing silane compound (D1-2), if contained in an excessively small ratio to a dialkoxysilyl-containing silane compound (D1-1), may cause an insufficient hair style retentivity and/or an insufficient smooth touch.

(Production Processes of Urethane-Modified Acrylic Polymers)

A method for producing urethane-modified acrylic polymers is not particularly limited, as long as being a method that can produce a urethane-modified acrylic polymer having a structure in which the above-mentioned acrylic polymer chain and the above-mentioned urethane polymer chain are bonded with each other through a Si-O bond-containing linkage segment. The production method can be selected suitably from among known methods. Among such methods, the method for producing vinyl-urethane copolymers described in WO 2005/054341 A1 is preferred. Specifically, an exemplary production method for producing urethane-modified acrylic polymers is a method that includes following Step (X) and Step (Y):

Step (X) of preparing an aqueous dispersion or aqueous solution of a urethane polymer (C); and
Step (Y) of, polymerizing an acrylic monomer (A) in the aqueous dispersion or aqueous solution of the urethane polymer (C), and preparing a urethane-modified acrylic polymer using a compound (B1) and/or a compound (B2) before the polymerization reaction, during the polymerization reaction, or after the polymerization reaction, wherein the urethane-modified acrylic polymer has a structure in which the above-mentioned urethane polymer chain is bonded to the above-mentioned acrylic polymer chain through a Si-O bond-containing linkage segment.

Step (Y) herein can be at least one step selected from following Steps (Y1-a), (Y1-b), (Y1-c), and (Y1-d):

Step (Y1-a):polymerizing an acrylic polymer (A) simultaneously with a reaction with a compound (B1) and/or a compound (B2) in the aqueous dispersion or aqueous solution of the urethane polymer (C), to yield a urethane-modified acrylic polymer in which the Si-O bond-containing linkage segment is a low-molecular-weight or high-molecular-weight Si-O bond-containing linkage segment;
Step (Y1-b):polymerizing an acrylic polymer (A) in the aqueous dispersion or aqueous solution of the urethane polymer (C), and thereafter reacting the products with a compound (B1) and/or a compound (B2), to yield a urethane-modified acrylic polymer in which the Si-O bond-containing linkage segment is a low-molecular-weight or high-molecular-weight Si-O bond-containing linkage segment;
Step (Y1-c):carrying out a reaction using a hydrolyzable silicon-containing group of a compound (B1) and/or a

compound (B2) in the aqueous dispersion or aqueous solution of the urethane polymer (C), and thereafter polymerizing an acrylic polymer (A) simultaneously with a reaction using an ethylenically unsaturated bond-containing group and/or a mercapto group of the compound (B1) and/or the compound (B2), to yield a urethane-modified acrylic polymer in which the Si-O bond-containing linkage segment is a low-molecular-weight or high-molecular-weight Si-O bond-containing linkage segment; and

Step (Y1-d) of carrying out a reaction using a hydrolyzable silicon-containing group of a compound (B1) and/or a compound (B2) in the aqueous dispersion or aqueous solution of the urethane polymer (C), and thereafter carrying out the polymerization of an acrylic polymer (A) simultaneously with a reaction using an ethylenically unsaturated bond-containing group and/or a mercapto group of the compound (B1) and/or the compound (B2) and further simultaneously with a reaction using another additional portion of the compound (B1) and/or another additional portion of the compound (B2), to yield a urethane-modified acrylic polymer in which the Si-O bond-containing linkage segment is a low-molecular-weight or high-molecular-weight Si-O bond-containing linkage segment.

In particular, when the urethane-modified acrylic polymer is a urethane-modified acrylic ternary copolymer, Step (Y) can be "the step of, in the aqueous dispersion or aqueous solution of the urethane polymer (C), carrying out the polymerization of an acrylic monomer (A), and using at least one of a compound (B1) and a compound (B2) and using a silane compound (D) simultaneously or separately before the polymerization reaction, during the polymerization reaction, and after the polymerization reaction, to thereby yield a urethane-modified acrylic polymer in which the urethane polymer chain is bonded to the acrylic polymer chain through a silicone polymer chain as a Si-O bond-containing linkage segment (a high-molecular-weight Si-O bond-containing linkage segment)". Specifically, Step (Y) can be at least one step selected from following Steps (Y2-a), (Y2-b), and (Y2-c) :

Step (Y2-a) of carrying out the hydrolysis or condensation of a silane compound (D) in the aqueous dispersion or aqueous solution of the urethane polymer (C), thereafter carrying out the polymerization of an acrylic monomer (A), and further using a compound (B1) and/or a compound (B2) in at least one period to thereby yield a urethane-modified acrylic polymer in which the Si-O bond-containing linkage segment is a silicone polymer chain, where the at least one period is selected from before the hydrolysis or condensation reaction, during the hydrolysis or condensation reaction, after the hydrolysis or condensation reaction or before the polymerization reaction, during the polymerization reaction, and after the polymerization reaction;

Step (Y2-b) of carrying out the hydrolysis or condensation of a silane compound (D) simultaneously with the polymerization of an acrylic monomer (A) in the aqueous dispersion or aqueous solution of the urethane polymer (C), and using a compound (B1) and/or a compound (B2) in at least one period to thereby yield a urethane-modified acrylic polymer in which the Si-O bond-containing linkage segment is a silicone polymer chain, where the at least one period is selected from before the hydrolysis or condensation reaction and the polymerization reaction, during the hydrolysis or condensation reaction and the polymerization reaction, and after the hydrolysis or condensation reaction and the polymerization reaction; and

Step (Y2-c) of carrying out the polymerization of an acrylic monomer (A) in the aqueous dispersion or aqueous solution of the urethane polymer (C), thereafter carrying out the hydrolysis or condensation of a silane compound (D), and using a compound (B1) and/or a compound (B2) in at least one period to thereby yield a urethane-modified acrylic polymer in which the Si-O bond-containing linkage segment is a silicone polymer chain, where the at least one period is selected from before the polymerization reaction, during the polymerization reaction, after the polymerization reaction or before the hydrolysis or condensation reaction, during the hydrolysis or condensation reaction, and after the hydrolysis or condensation reaction.

Processes for producing urethane-modified acrylic polymers according to the present invention include a process that includes Step (X) and Step (Y) [e.g., Step (Y1-a), Step (Y1-b), Step (Y1-c), and/or Step (Y1-d)] as a production process for producing a urethane-modified acrylic binary copolymer; a process that includes Step (X) and Step (Y) [e.g., Step (Y1-a), Step (Y1-b), Step (Y1-c), and/or Step (Y1-d)] as a process for producing a urethane-modified acrylic ternary copolymer; a process that includes Step (X) and Step (Y2-a), Step (X) and Step (Y2-b), or Step (X) and Step (Y2-c); and a process that includes any combination of these steps.

When an importance is placed on the hair styling capability and hair style retentivity, Step (X) is preferably following Step (X1); and, when an importance is placed on the natural feeling and smooth touch, Step (X) is preferably following Step (X2):

Step (X1) of preparing a urethane polymer (C) in a known or common organic solvent as a solvent, thereafter distilling off the organic solvent to give a reaction mixture, and dispersing or dissolving the reaction mixture in water, to thereby yield an aqueous dispersion or aqueous solution of the urethane polymer (C); and

Step (X2) of preparing a urethane polymer (C) in an acrylic monomer (A) as a solvent to give a reaction mixture,

and dispersing or dissolving the reaction mixture in water, to thereby yield an aqueous dispersion or aqueous solution of the urethane polymer (C) which contains the acrylic monomer (A).

A high-molecular-weight urethane polymer (C) can be prepared by preparing the urethane polymer (C) in an organic solvent as a solvent according to Step (X1). This enables the production of a urethane-modified acrylic copolymer containing such a high-molecular-weight urethane polymer (C), to thereby yield a hair cosmetic that exhibits a furthermore superior hair styling capability and a furthermore superior hair style retentivity.

In contrast, a urethane-modified acrylic polymer can be furthermore efficiently produced without the need of removing solvents (e.g., organic solvents such as ketones and pyrrolidones) in contrast to common processes, by preparing a urethane polymer (C) according to Step (X2), because Step (X2) employs an acrylic monomer (A) as a solvent. Additionally, this step eliminates the need of disposing organic solvents as waste fluids, is thereby superior also from the viewpoint of environment, and can reduce the cost.

A process to disperse or dissolve a urethane polymer (C) in water in Step (X) can be suitably selected from among, for example, the processes described in WO 2005/054341 A1. An exemplary process is the process of mixing the urethane polymer (C) with water, if necessary, using a basic compound and/or a dispersing agent (e.g., an emulsifier or a surfactant), to thereby disperse or dissolve the urethane polymer (C) in water. Exemplary preferred basic compounds for use herein include alkali metal hydroxides such as sodium hydroxide and potassium hydroxide; ammonia; triethanolamine, tripropanolamine, triisopropanolamine, tributanolamine, tripentanolamine, triisopentanolamine, trihexanolamine, and 2-methylpropanolamine.

A process for the polymerization of an acrylic polymer (A) in Step (Y) is not particularly limited and can be selected from among known or common polymerization processes of acrylic monomers. Among them, the polymerization process disclosed in WO 2005/054341 A1 is preferred. A process for carrying out the hydrolysis reaction or condensation reaction (condensation polymerization) of a silane compound (D) is not particularly limited, and can be selected from among known or common processes for carrying out hydrolysis reactions of silicone compounds or known or common processes for carrying out condensation reactions of silicone compounds. Among them, the processes for carrying out a hydrolysis reaction or a condensation reaction disclosed in WO 2005/054341 A1 are preferred. In this connection, compounds (B1) and compounds (B2) can be subjected to polymerization and/or hydrolysis or condensation by the same procedure as in the acrylic monomer (A) and the silane compound (D). Details of this can be found in WO 2005/054341 A1.

The ratio by mass of acrylic polymer chains (except for hydrolyzable silicon-containing groups) to urethane polymer chains (except for hydrolyzable silicon-containing groups) in the urethane-modified acrylic polymer can be selected within a range of 0.02 or more and 10 or less.

Specifically, when an importance is placed on the hair styling capability and hair style retentivity, the urethane-modified acrylic polymer is preferably urethane-rich (abundant in urethane chains), and the ratio by mass of acrylic polymer chains (except for hydrolyzable silicon-containing groups) to urethane polymer chains (except for hydrolyzable silicon-containing groups) can be selected within a range of 0.02 or more and less than 3. In contrast, when an importance is placed on the natural feeling and smooth touch, the urethane-modified acrylic polymer is preferably acrylic-rich (abundant in acrylic chains), and the ratio by mass of acrylic polymer chains (except for hydrolyzable silicon-containing groups) to urethane polymer chains (except for hydrolyzable silicon-containing groups) can be selected within a range of 3 or more and 10 or less.

The proportions of respective components [e.g., urethane polymers (C), acrylic monomers (A), compounds (B1), compounds (B2), and silane compounds (D)] in urethane-modified acrylic polymers are not particularly limited and can be suitably selected within the proportions disclosed in WO 2005/054341 A1.

Specifically, when the urethane-modified acrylic polymer is a urethane-modified acrylic binary copolymer, the ratio (equivalent ratio) of hydrolyzable silicon-containing groups in the urethane polymer (C) to hydrolyzable silicon-containing groups in the compound (B1) and/or the compound (B2) may be selected within a range of more than 0.01 and 20 or less, and preferably from 0.05 to 10.

On the other hand, when the urethane-modified acrylic polymer is a urethane-modified acrylic ternary copolymer, the ratio of the urethane polymer (C) to the silane compound (D) may be selected within such a range that the ratio (equivalent ratio) of hydrolyzable silicon-containing groups in the urethane polymer (C) to hydrolyzable silicon-containing groups in the silane compound (D) is 0.001 or more, for example from 0.001 to 10, and preferably from 0.008 to 5. The ratio of the compound (B1) and/or compound (B2) to the silane compound (D) may be selected within such a range that the ratio (equivalent ratio) of hydrolyzable silicon-containing groups in the compound (B1) and/or compound (B2) to hydrolyzable silicon-containing groups in the silane compound (D) is 0.0001 or more, for example from 0.002 to 100, and preferably from 0.01 to 10.

Whenever the urethane-modified acrylic polymer is a urethane-modified acrylic binary copolymer or a urethane-modified acrylic ternary copolymer, the ratio of the acrylic monomer (A) to the compound (B1) and/or compound (B2) may be selected within such a range that the ratio (equivalent ratio) of ethylenically unsaturated bonds in the acrylic

monomer (A) to ethylenically unsaturated bond-containing groups in the compound (B1) and/or mercapto groups in the compound (B2) is from 0.2 to 2500, preferably from 0.6 to 500, and more preferably from 1 to 100.

Thus, urethane-modified acrylic polymers can be prepared herein by, in the aqueous dispersion or aqueous solution of the urethane polymer (C), carrying out the polymerization of an acrylic monomer (A), and using a compound (B1) and/or compound (B2) and a silane compound (D) simultaneously or separately in at least one period selected from before the polymerization reaction, during the polymerization reaction, and after the polymerization reaction. The urethane-modified acrylic polymers can therefore be prepared according to the form of an aqueous dispersion or aqueous solution. The product urethane-modified acrylic polymers produced by the method herein can be used as intact as components of hair cosmetics.

(Hair Cosmetics)

Hair cosmetics according to the present invention contain the urethane-modified acrylic polymers as described above and can thereby exhibit hair styling capability, natural finishing capability, and hair style retentivity respectively at superior levels. Among them, hair cosmetics that contain a urethane-modified acrylic ternary copolymer as the urethane-modified acrylic polymer can exhibit hair styling capability, natural finishing capability, and hair style retentivity at furthermore superior levels and can develop a smooth touch at a furthermore superior level. Additionally, control of components of the urethane-modified acrylic polymer gives hair cosmetics that can exhibit desired properties typified by hair styling capability, hair style retentivity, natural finishing capability, and hair style retentivity respectively at superior levels.

Hair cosmetics according to the present invention, if used, enable the hair to set with no or substantially no sticky feeling during a duration from the application of the hair cosmetic to the hair to drying of the applied hair cosmetic and enable the hair in a set hair style to comb with no or substantially no flaking. Additionally, the hair cosmetics, if applied to the hair, can be washed out from the hair satisfactorily, and this enables the hair to shampoo easily and satisfactorily.

Therefore, the hair cosmetics, if used, enable the hair to set with a natural feeling and a pliable and smooth touch without no sticky feeling during a duration from the application of the hair cosmetic to the hair to drying of the applied hair cosmetic and enable the hair to set to a predetermined hair style highly satisfactorily. They further enable the set hair to comb without flaking and enable the set hair style, such as curled hair, to retain even at high humidity. Additionally, they can be washed out satisfactorily, and this enables the hair to shampoo satisfactorily.

Hair cosmetics according to the present invention preferably contain the urethane-modified acrylic polymers in the form of an aqueous dispersion or aqueous solution. Such urethane-modified acrylic polymers can be used as hair styling components, and preferably as film-formable polymer components, in the hair cosmetics according to the present invention. The content of urethane-modified acrylic polymers in the hair cosmetics is not particularly limited but can be selected within a range of 0.1 to 10 percent by mass, and preferably 0.5 to 5.0 percent by mass, in terms of solids content, based on the total amount of the hair cosmetic. A hair cosmetic, if containing a urethane-modified acrylic polymer in a content of less than 0.1 percent by mass based on the total amount of the hair cosmetic, may exhibit an insufficient hair styling capability. In contrast, a hair cosmetic, if containing a urethane-modified acrylic polymer in a content of more than 10 percent by mass based on the total amount of the hair cosmetic, may be difficult to set the hair in a natural finish, may cause a coarse/stiff feeling and flaking after the hair setting.

The hair cosmetics according to the present invention may further contain known hair styling components according to necessity, in addition to the urethane-modified acrylic polymers. Exemplary hair styling components include waxes such as beeswax, lanolin, and candelilla wax; and film-formable polymer components such as polyvinylpyrrolidones, sodium polyacrylates, and polyvinylpyrrolidone-polyvinyl acetate copolymers.

As appropriate, the hair cosmetics may further contain known components generally used in skin external preparations such as cosmetics and pharmaceutical drugs within ranges not adversely affecting the advantages of the present invention. Exemplary components herein include powder components, liquid oils (fatty oils), solid fats (fats), waxes, hydrocarbon oils, higher fatty acids, higher alcohols, esters, silicone components, anionic surfactants, cationic surfactants, amphoteric surfactants, nonionic surfactants, humectants, water-soluble polymers, thickeners, film-forming agents, ultraviolet absorbers, metal-ion sequestering agents, lower alcohols, polyhydric alcohols, saccharides, amino acids, organic amines, polymer emulsions, pH adjusters, skin nutrients, vitamins, antioxidants, antioxidant assistants, perfumes, and water. These components will be illustrated in detail below. As used herein a "POE" refers to a "polyoxyethylene"; a "POP" refers to a "polyoxypropylene"; a "POE-POP" refers to a polyoxyethylene-polyoxypropylene copolymer; and a "POP-POE" refers to a polyoxypropylene-polyoxyethylene copolymer. There are some cases where one compound is categorized as two or more different components. Each of compounds can be suitably categorized as one or more components according typically to the purpose of use.

Exemplary powder components include inorganic powders such as talc, kaolin, mica, sericite, muscovite, phlogopite, synthetic mica, lepidolite, biotite, permiculite, magnesium carbonate, calcium carbonate, aluminum silicate, barium silicate, calcium silicate, magnesium silicate, strontium silicate, metal tungstates, magnesium, silica, zeolite,

barium sulfate, calcined calcium sulfate (calcined gypsum), calcium phosphate, fluoroapatite, hydroxyapatite, ceramic powders, metal soaps (e.g., zinc myristate, calcium palmitate, and aluminum stearate), and boron nitride; organic powders such as polyamide resin powder (nylon powder), polyethylene powder, poly(methyl methacrylate) powder, polystyrene powder, styrene/acrylic acid copolymer powder, benzoguanamine resin powder, poly(ethylene tetrafluoride) powder, and cellulose powder; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and yellow ocher; inorganic black pigments such as black iron oxide and low-dimensional titanium oxide; inorganic purple pigments such as mango violet (manganese violet) and cobalt violet; inorganic green pigments such as chromium oxide, chromium hydroxide, and cobalt titanate; inorganic blue pigments such as ultramarine and Prussian blue; pearl pigments such as titanium oxide-coated mica, titanium oxide-coated bismuth oxychloride, titanium oxide-coated talc, colored titanium oxide-coated mica, bismuth oxychloride, and fish scale; metal powder pigments such as aluminum powder and copper powder; zirconium lakes, barium lakes, aluminum lakes, and other organic pigments, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, and other organic pigments, as well as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and natural colorants such as chlorophyll and β-carotene.

Exemplary liquid oils include avocado oil, camellia oil, turtle oil, macadamia nut oil, corn oil, mink oil, olive oil, rapeseed oil, yolk oil, sesame oil, persic oil, wheat germ oil, sasanqua oil, castor oil, linseed oil, safflower oil, cotton seed oil, perilla oil, soybean oil, peanut oil, tea seed oil, torreya oil, rice bran oil, Chinese tung oil, Japanese tung oil, jojoba oil, germ oil, and triglycerols.

Exemplary solid fats include cacao fat, coconut oil, horse fat, hydrogenated coconut oil, palm oil, beef tallow, sheep tallow, hydrogenated beef tallow, palm kernel oil, lard, beef bone fat, Rhus succedanea (Japan tallow) kernel oil, hydrogenated oils, beef foot tallow, Japan tallow, and hydrogenated castor oil.

Exemplary waxes include beeswax, candelilla wax, cotton wax, carnauba wax, bayberry wax, insect wax, spermaceti, montan wax, rice bran wax, lanolin, kapok wax, lanolin acetate, liquid lanolin, sugarcane wax, isopropyl lanolin fatty acids, hexyl laurate, hydrogenated lanolin, jojoba wax, lanolin wax, shellac wax, POE lanolin alcohol ether, POE lanolin alcohol acetate, POE cholesterol ether, lanolin fatty acid poly(ethylene glycol)s, and POE hydrogenated lanolin alcohol ether.

Exemplary hydrocarbon oils include liquid paraffin, ozokerite, squalane, pristane, paraffin, ceresin, squalene, petrolatum (Vaseline), and microcrystalline wax.

Exemplary higher fatty acids include lauric acid, myristic acid, palmitic acid, stearic acid, behenic acid, oleic acid, undecylenic acid, tall oil acids, isostearic acid, linoleic acid, linolenic acid, eicosapentaenoic acid (EPA), and docosahexaenoic acid (DHA).

Exemplary higher alcohols include linear alcohols such as lauryl alcohol, cetyl alcohol, stearyl alcohol, behenyl alcohol, myristyl alcohol, oleyl alcohol, and cetostearyl alcohol; and branched alcohols such as monostearyl glyceryl ether (batyl alcohol), 2-decyltetradecynol, lanolin alcohol, cholesterols, phytosterols, hexyldodecanol, isostearyl alcohol, and octyldodecanol.

Of esters, preferred are synthetic ester oils. Exemplary synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid esters, N-alkyl glycol monoisostearates, neopentyl glycol dicaprate, diisostearyl malate, glyceryl di-2-heptylundecanoate, trimethylolpropane tri-2-ethylhexanoate, trimethylolpropane triisostearate, pentaerythritol tetra(2-ethylhexanoate), glyceryl tri(2-ethylhexanoate), glyceryl trioctanoate, glyceryl tiriisopalmitate, trimethylolpropane triisostearate, cetyl 2-ethylhexanoate, 2-ethylhexyl palmitate, glyceryl trimyristate, glyceryl tri(2-heptylundecanoate), castor oil fatty acid methyl esters, oleyl oleate, acetoglyceride (glyceryl monoacetate), 2-heptylundecyl palmitate, diisobutyl adipate, 2-octyldodecyl N-lauroyl-L-glutamate, di-2-heptylundecyl adipate, ethyl laurate, di(2-ethylhexyl) sebacate, 2-hexyldecyl myristate, 2-hexyldecyl palmitate, 2-hexyldecyl adipate, diisopropyl sebacate, 2-ethylhexyl succinate, and triethyl citrate.

Silicone components for use herein are preferably oily silicone components (silicone oils) but may also be non-oily silicone components. Exemplary silicone components include chain polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcyclohexasiloxane; silicone resins having three-dimensional network structures; silicone rubbers; and modified polysiloxanes such as amino-modified polysiloxanes, polyether-modified polysiloxanes, alkyl-modified polysiloxanes, and fluorine-modified polysiloxanes.

Exemplary anionic surfactants include fatty acid soaps such as sodium laurate and sodium palmitate; higher alkyl sulfuric ester salts such as sodium lauryl sulfate and potassium lauryl sulfate; alkyl ether sulfuric ester salts such as triethanolamine POE lauryl ether sulfate and sodium POE lauryl ether sulfate; N-acyl sarcosine salts such as sodium

lauroyl sarcosine; higher fatty acid amide sulfonate salts such as sodium N-myristoyl N-methyltaurine, sodium coconut oil fatty acid methyltaurides, and sodium lauryl methyltauride; phosphoric ester salts such as sodium POE oleyl ether phosphate and POE stearyl ether phosphate; sulfosuccinic acid salts such as sodium di(2-ethylhexyl) sulfosuccinate, sodium monolauroyl monoethanolamide polyoxyethylene sulfosuccinate, and sodium lauryl polypropylene glycol sulfosuccinate; alkylbenzenesulfonic acid salts such as sodium linear dodecylbenzenesulfonate, triethanolamine linear dodecylbenzenesulfonate, and linear dodecylbenzenesulfonic acid; higher fatty acid ester sulfate salts such as sodium hydrogenated cocoate glyceryl sulfate; N-acylglutamic acid salts such as monosodium N-lauroyl glutamate, disodium N-stearoyl glutamate, and monosodium N-myristoyl L-glutamate; sulfated oils such as sulfonated caster oil; POE alkyl ether-carboxylic acids; POE alkyl allyl ether-carboxylic acid salts; $\alpha$-olefinsulfonic acid salts; higher fatty acid estersulfonic acid salts; secondary alcohol sulfuric ester salts; higher fatty acid alkylolamide sulfuric ester salts; sodium lauroyl monoethanolamidosuccinate; ditriethanolamine N-palmitoylaspartate; and sodium caseinate.

Exemplary cationic surfactants include alkyltrimethylammonium salts such as stearyltrimethylammonium chloride and lauryltrimethylammonium chloride; alkylpyridinium salts such as cetylpyridinium; dialkyldimethylammonium salts such as distearyldimethylammonium chloride; dicocoylethyl hydroxyethylmonium methosulfate; poly(N,N'-dimethyl-3,5-methylenepiperidinium) chloride; alkyl quaternary ammonium salts; alkyldimethylbenzylammonium salts; alkylisoquinolinium salts; dialkylmorpholinium salts; POE alkylamines; alkylamine salts; polyamine fatty acid derivatives; amyl alcohol fatty acid derivatives; benzalkonium chloride; and benzethonium chloride.

Exemplary amphoteric surfactants include imidazoline amphoteric surfactants such as sodium 2-undecyl-N,N,N-(hydroxyethylcarboxymethyl)-2-imidazoline and disodium 2-cocoyl-2-imidazolinium hydroxide-1-carboxyethyloxy; and betaine surfactants such as 2-heptadecyl-N-carboxymethyl-N-hydroxyethylimidazolinium betaine, lauryldimethylaminoacetic acid betaine, alkylbetaines, amido-betaines, and sulfobetaines.

Nonionic surfactants include lipophilic nonionic surfactants and hydrophilic nonionic surfactants. Exemplary lipophilic nonionic surfactants include sorbitan fatty acid esters such as sorbitan monooleate, sorbitan monoisostearate, sorbitan monolaurate, sorbitan monoplamitate, sorbitan monostearate, sorbitan sesquioleate, sorbitan trioleate, diglyceryl sorbitan penta-2-ethylhexanoate, and diglyceryl sorbitan tetra-2-ethylhexanoate; glyceryl/polyglyceryl fatty acids such as glyceryl mono(cotton seed oil fatty acid) esters, glyceryl monoerucate, glyceryl sesquioleate, glyceryl monostearate, glyceryl $\alpha,\alpha'$-oleate pyroglutamate, and glyceryl monostearate malate; propylene glycol fatty acid esters such as propylene glycol monostearate; hydrogenated caster oil derivatives; and alkyl ethers of glycerol.

Exemplary hydrophilic nonionic surfactants include POE sorbitan fatty acid esters such as POE sorbitan monooleate, POE sorbitan monostearate, and POE sorbitan tetraoleate; POE sorbitol fatty acid esters such as POE sorbitol monolaurate, POE sorbitol monooleate, POE sorbitol pentaoleate, and POE sorbitol monostearate; POE glyceryl fatty acid esters such as POE glyceryl monostearate, POE glyceryl monoisostearate, POE glyceryl triisostearate, and POE glyceryl monooleate; POE fatty acid esters such as POE distearate, POE monooleate, POE dioleate, and ethylene glycol distearate; POE alkyl ethers such as POE lauryl ether, POE oleyl ether, POE stearyl ether, POE behenyl ether, POE 2-octyldodecyl ether, and POE cholestanol ether; Pluronic surfactants such as Pluronic; POE-POP alkyl ethers such as POE POP cetyl ether, POE POP 2-decyltetradecyl ether, POE POP monobutyl ether, POE POP hydrogenated lanolin, and POE POP glyceryl ether; tetra-POE tetra-POP ethylenediamine condensates such as Tetronics; POE caster oil or POE hydrogenated caster oil derivatives such as POE caster oil, POE hydrogenated caster oil, POE hydrogenated caster oil monoisostearate, POE hydrogenated caster oil triisostearate, POE hydrogenated caster oil monopyroglutamate monoisostearate, and POE hydrogenated caster oil maleate; POE beeswax/lanolin derivatives such as POE sorbitol beeswax; alkanolamides such as coconut oil fatty acid diethanolamide, lauroyl monoethanolamide, and fatty acid isopropanolamides; POE propylene glycol fatty acid esters; POE alkylamines; POE fatty acid amides; sucrose fatty acid esters; alkylethoxydimethylamine oxides; and trioleyl phosphate.

Exemplary humectants include poly(ethylene glycol)s, propylene glycol, glycerol, 1,3-butylene glycol, xylitol, sorbitol, maltitol, chondroitin sulfate, hyaluronic acid, mucoitinsulfuric acid, charonic acid, atelocollagen, cholesteryl 12-hydroxystearate, sodium lactate, bile salts, dl-pyrrolidonecarboxylic acid salts, short-chain soluble collagen, diglyceryl (EO)PO adducts [diglyceryl (ethylene oxide) propylene oxide adducts], Rosa roxburghii extract, yarrow extract, and melilot extract.

Exemplary water-soluble polymers include naturally occurring water-soluble polymers, semisynthetic water-soluble polymers, and synthetic water-soluble polymers. Exemplary naturally occurring water-soluble polymers include polymers of vegetable origin, such as gum arabic, tragacanth gum, galactan, guar gum, carob gum, karaya gum, carrageenan, pectin, agar, quince seed (marmelo), algae colloid (brown algae extract), starches (starches made typically from rice, corn, potatoes and wheat), and glycyrrhizic acid; polymers of microbial origin, such as xanthane gum, dextran, succinoglucan, and pullulan; and polymers of animal origin, such as collagen, casein, albumin, and gelatin. Exemplary semisynthetic water-soluble polymers include starch-based polymers such as carboxymethyl starch and methylhydroxypropyl starch; cellulose-based polymers such as methylcellulose, ethylcellulose, methylhydroxypropylcellulose, hydroxyethylcellulose, cellulose sulfate sodium, hydroxypropylcellulose, carboxymethylcellulose, carboxymethylcellulose sodium, crystalline cellulose, and powdered cellulose; alginic acid-based polymers such as sodium alginate and alginic

acid propylene glycol ester; and cationized derivatives of naturally occurring polymers, such as cationized guar gum, cationized locust bean gum, cationized celluloses, and cationized starches. Exemplary synthetic water-soluble polymers include vinyl polymers such as poly(vinyl alcohol)s, poly(vinyl methyl ether)s, polyvinylpyrrolidones, carboxyvinyl polymers, and poly(vinyl acetate)s; polyoxyethylene polymers such as poly(ethylene glycol)s, poly(propylene glycol)s, polyoxyethylene/polyoxypropylene copolymers, polyoxyethylene/polyoxybutylene copolymers, and poly(ethylene glycol)/polyoxyethylene alkyl ether/hexamethylene diisocyanate copolymers; acrylic polymers such as sodium polyacrylates, poly(ethyl acrylate)s, polyacrylamides, acrylic acid/alkyl methacrylate copolymers, alkyl acrylate/alkyl methacrylate/polyoxyethylene alkyl ether copolymers, and crosslinkable copolymers of N,N-dimethylacrylamide-2-acrylamide-2-methylpropanesulfonic acid salts; polyethyleneimines; and cationic polymers.

Exemplary thickeners include gum arabic, carrageenan, karaya gum, tragacanth gum, carob gum, quince seed (marmelo), casein, dextrin, gelatin, sodium pectate, sodium alginate, methylcellulose, ethylcellulose, carboxymethylcellulose (CMC), hydroxyethylcellulose, hydroxypropylcellulose, poly(vinyl alcohol)s (PVA), vinyl methyl ether/butyl maleate copolymers (PVM), polyvinylpyrrolidones (PVP), sodium polyacrylates, carboxyvinyl polymers, acrylic acid/alkyl methacrylate copolymers, poly(urethane-urea) derivatives, locust bean gum, guar gum, tamarind gum, dialkyldimethylammonium sulfate cellulose, xanthan gum, magnesium aluminum silicate, bentonite, hectorite, AlMg silicate (Veegum; magnesium aluminum silicate), Laponite, and silicic anhydride.

Exemplary film-forming agents include vinyl polymers such as vinylpyrrolidone/vinyl alcohol copolymers and polyvinylpyrrolidones; acrylic polymers such as alkyl acrylate/diacetoneacrylamide copolymers, vinylpyrrolidone/N,N-dimethyl-antinioethyl methacrylate copolymers, dimethyldiallylammonium chloride/acrylamide copolymers, vinylpyrrolidone/N,N'-dimethylaminoethyl methacrylate/alkyl acrylate/tripropylene glycol diacrylate copolymers, N-methacryloyloxyethyl N,N-dimethylammonium-α-N-methylcarboxy-betaine/alkyl methacrylate copolymers; and urethane polymers.

Exemplary ultraviolet absorbers include benzoic acid ultraviolet absorbers such as p-aminobenzoic acid (hereinafter also referred to as "PABA"), PABA monoglyceryl ester, N,N-dipropoxy-PABA ethyl ester, N,N-diethoxy-PABA ethyl ester, N,N-dimethyl-PABA ethyl ester, and N,N-dimethyl-PABA butyl ester; anthranilic acid ultraviolet absorbers such as homomenthyl-N-acetyl anthranilate; salicylic acid ultraviolet absorbers such as amyl salicylate, menthyl salicylate, homomenthyl salicylate, octyl salicylate, phenyl salicylate, benzyl salicylate, and p-isopropanolphenyl salicylate; cinnamic acid ultraviolet absorbers such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, and glyceryl mono-2-ethylhexanoyl-di-p-methoxycinnamate; 3-(4'-methylbenzylidene)-d,1-camphor and 3-benzylidene-d,1-camphor; 2-phenyl-5-methylbenzoxazol; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one, and dimorpholinopyridazinone.

Exemplary metal-ion sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediaminehydroxyethyltriacetate, and malic acid.

Exemplary lower alcohols include ethanol, propanol, isopropyl alcohol, isobutyl alcohol, and t-butyl alcohol.

Exemplary polyhydric alcohols include dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols such as glycerol and trimethylolpropane; tetrahydric alcohols such as 1,2,6-hexanetriol and pentaerythritol; pentahydric alcohols such as xylitol; hexahydric alcohols such as sorbitol and mannitol; polyhydric alcohol polymers such as diethylene glycol, dipropylene glycol, triethylene glycol, poly(propylene glycol)s, tetraethylene glycol, diglycerol, poly(ethylene glycol)s, triglycerol, tetraglycerol, and polyglycerols; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate,

propylene glycol monoethyl ether acetate, propylene glycol monopropyl ether acetate, and propylene glycol monophenyl ether acetate; glycerol monoalkyl ethers such as chimyl alcohol (1-hexadecyl glycerol ether), selachyl alcohol (1-(9-octadecenyl) glycerol ether), and batyl alcohol (1-octadecyl glycerol ether); sugar alcohols such as sorbitol, maltitol, maltotriose, mannitol, sucrose, erythritol, glucose, fructose, starch-decomposed sugars, maltose, xylitose, and alcohols prepared by the reduction of starch-decomposed sugars); Glysolid; tetrahydrofurfuryl alcohol; POE-tetrahydrofurfuryl alcohol; POP butyl ether; POP-POE butyl ether; tripolyoxypropylene glyceryl ether; POP glyceryl ether; POP glyceryl ether phosphate; POP-POE pentane erythritol ether, and polyglycerols.

Saccharides include monosaccharides, oligosaccharides, and polysaccharides. Exemplary monosaccharides include trioses such as D-glyceraldehyde and dihydroxyacetone; tetroses such as D-erythrose, D-erythrulose, D-threose, and erythritol; pentoses such as L-arabinose, D-xylose, L-lyxose, D-arabinose, D-ribose, D-ribulose, D-xylulose, L-xylulose, and xylitol; hexoses such as D-glucose, D-talose, D-psicose, D-galactose, D-fructose, L-galactose, L-mannose, and D-tagatose; heptoses such as aldoheptoses and heptuloses (ketoheptoses); octoses such as octuloses; deoxysugars such as 2-deoxy-D-ribose, 6-deoxy-L-galactose, and 6-deoxy-L-mannose; amino sugars such as D-glucosamine, D-galactosamine, sialic acids, aminouronic acids, and muramic acid; and uronic acids such as D-glucuronic acid, D-mannuronic acid, L-guluronic acid, D-galacturonic acid, and L-iduronic acid. Exemplary oligosaccharides include sucrose, gentianose, umbelliferose, lactose, planteose, isolignoses, $\alpha,\alpha$-trehalose, raffinose, lignoses, umbellicine, stachyose, and verbascose and analogues thereof. Exemplary polysaccharides include cellulose, quince seed, chondroitin sulfuric acid, starch, galactan, dermatan sulfate, glycogen, gum arabic, heparan sulfate, hyaluronic acid, gum tragacanth, keratan sulfate, chondroitin, xanthan gum, mucoitin sulfuric acid, guar gum, dextran, keratosulfate, locust bean gum, succinoglucan, and charonic acid.

Amino acids include amino acids and amino acid derivatives. Exemplary amino acids include neutral amino acids such as threonine and cysteine; basic amino acids such as hydroxylysine; and arginine and lysine. Exemplary amino acid derivatives include sodium acylsarcosinates (e.g., sodium lauroylsarcosinate), acylglutaminate salts, sodium acyl $\beta$-alanine, glutathione, and pyrolidonecarboxylic acid.

Exemplary organic amines include monoethanolamine, diethanolamine, triethanolamine, morpholine, triisopropanolamine, 2-amino-2-methyl-1,3-propanediol, and 2-amino-2-methyl-1-propanol.

Exemplary polymer emulsions include acrylic resin emulsions, poly(ethyl acrylate) emulsions, acrylic resin solutions, poly(alkyl acrylate) emulsions, poly(vinyl acetate) emulsions, and natural rubber latices.

Exemplary pH adjusters include buffers such as lactic acid-sodium lactate, citric acid-sodium citrate, and succinic acid-sodium succinate.

Exemplary vitamins include vitamin A, vitamin B1, vitamin B2, vitamin B6, vitamin C, vitamin E, and derivatives of them, as well as pantothenic acid and derivatives thereof, and biotin.

Exemplary antioxidants include tocopherols, dibutylhydroxytoluene, butylhydroxyanisole, and gallic acid esters. Exemplary antioxidant assistants include phosphoric acid, citric acid, ascorbic acid, maleic acid, malonic acid, succinic acid, fumaric acid, kephalin, hexametaphosphate, phytic acid, and ethylenediaminetetraacetic acid.

Exemplary other components usable in the hair cosmetics include preservatives such as ethylparaben (ethyl p-hydroxybenzoate) and butylparaben (butyl p-hydroxybenzoate); anti-inflammatory agents such as glycyrrhizic acid derivatives, glycyrrhetic acid derivatives, salicylic acid derivatives, hinokitiol, zinc oxide, and allantoin; whitening agents such as placenta extract, creeping saxifrage extract, arbutin, vitamin C, magnesium ascorbyl phosphate, ascorbyl glucoside, and kojic acid; extracts including components extracted typically from phellodendron bark, Coptis root, Lithospermi Radix, peony root, swertia herb, birch, sage, loquat, ginseng, aloe, mallow, iris, grape, coix seed, sponge gourd, lily, saffron, cnidium rhizome, ginger, Hypericum erectum, Ononis spinosa, garlic, capsicum, citrus unshiu peel, Angelica root, and seaweed; activators such as royal jelly, photosensitizers, and cholesterol derivatives; blood circulation promoters such as nonylic acid valenylamide, benzyl nicotinate, $\beta$-butoxyethyl nicotinate, capsaicin, zingerone, cantharis tincture, ichthammol, tannic acid, $\alpha$-borneol, tocopherol nicotinate, inositol hexanicotinate, cyclandelate, cinnarizine, tolazoline, acetylcholine, verapamil, cepharanthin, and $\gamma$-orizanol; antiseborrheic agents such as sulfur and thianthol; antiinflammatory agents such as tranexamic acid, thiotaurine, and hypotaurine; other ingredients such as caffeine, tannin, verapamil, tranexamic acid and derivatives thereof, and extracts of crude drugs such as liquorice root, Chinese quince, and Pyrola japonica; and other drugs or agents such as tocopherol acetate, glycyrrhizic acid, glycyrrhetinic acid, and derivatives thereof, or salts of them.

The hair cosmetics according to the present invention may be in any formulation forms such as solutions, solubilized products, emulsions, liquid oils, powders, powder dispersions, gels, ointments, oil-water two-phase preparations, and oil-water-powder three-phase preparations. Additionally, they may be in any product forms such as aerosols, sprays, pumped sprays, mousses, sticks, and rollon products.

The hair cosmetics may be any forms of aqueous (waterborne) forms and oily forms. Regular hair cosmetics, if in an aqueous form, have a pH of 4.5 to 8.5. However, the hair cosmetics according to the present invention may have a pH of preferably 6 to 10, and more preferably 7 to 9. A hair cosmetic, if having a pH of lower than 6, may not retain its emulsion form. In contrast, a hair cosmetic, if having a pH of higher than 10, may cause damage to the hair.

The hair cosmetics according to the present invention are advantageously usable as hair styling agents (hair dressing agents) for setting hairs.

EXAMPLES

The present invention will be illustrated in further detail with reference to several examples below. It should be noted, however, that these examples are never construed to limit the scope of the present invention. All parts and percentages below are by mass, unless otherwise specified. In examples and comparative examples below, the amounts of respective components are indicated as amounts to prepare 100 parts by weight of a hair cosmetic (or a stock thereof), for the sake of convenience.

Hair cosmetics prepared according to examples and comparative examples were evaluated according to the following methods.

(Method for Evaluating Sticky Feeling During Duration After Application and Before Drying)

Each test sample (0.5 g) (each of hair cosmetics prepared according to examples and comparative examples) was applied to virgin black hair having a length of 20 cm and a weight of 2 g, and the hair was immediately combed and dried. Sticky feeling of the hair before drying was rated in a sensory test by special panelists of ten women. In the sensory test, the degree of sticky feeling until drying was sensorially scored according to the following scoring criteria, and the sticky feeling (freeness from sticky feeling) was evaluated according to the following evaluation criteria. In the scoring criteria, a higher score means a less sticky feeling during a duration after the application and before drying.

(Scoring Criteria)

Score 5: No or substantially no sticky feeling
Score 4: Little sticky feeling
Score 3: Slightly sticky feeling
Score 2: Somewhat sticky feeling
Score 1: Very sticky feeling

(Evaluation Criteria)

AAA: Total score of the ten women special panelists is 40 or more, and eight or more panelists evaluate as Score 5
AA: Total score of the ten women special panelists is 40 or more, and five or more panelists evaluate as Score 5
A: Total score of the ten women special panelists is 40 or more
B: Total score of the ten women special panelists is 30 or more and less than 40
C: Total score of the ten women special panelists is 20 or more and less than 30
D: Total score of the ten women special panelists is less than 20

(Method for Evaluating Hair Styling Capability)

Each test sample (0.5 g) (each of hair cosmetics prepared according to examples and comparative examples) was applied to virgin black hair having a length of 20 cm and a weight of 2 g, and the applied hair was immediately curled with a curler having a curling diameter of 2 cm, and the curled hair was dried at 50°C for one hour. The length of the curled hair strand was measured, and this length was defined as an initial length (L0).
Next, a load of 60 g was applied to the tip of hair for 15 minutes, the load was then removed, a tick mark of the tip of hair at the time when the length of the hair strand became constant was read out to measure the length of the hair strand, and this length was defined as a length after loading (L1).
The length of the hair strand is the maximum diameter of the curl when the hair strand remains being curled, and it is the maximum length from an end of the hair near to the hair root (e.g., the length between an end near to hair root and another end near to hair tip) when the curled hair is partially or fully uncurled.
A curling-memory rate of each sample was calculated according to the following equation, and the hair styling capability was evaluated according to the following evaluation criteria:

$$\text{Curling-memory rate (\%)} = \{(20-L1)/(20-L0)\} \times 100$$

A sample has higher curling retentivity, higher elasticity, and higher hair styling capability, when it has a higher curling-memory rate approaching 100%.

(Evaluation Criteria)

AAA: Curling-memory rate is 98% or more
AA: Curling-memory rate is 95% or more
A: Curling-memory rate is 90% or more
B: Curling-memory rate is 70% or more and less than 90%
C: Curling-memory rate is 50% or more and less than 70%
D: Curling-memory rate is less than 50%

(Method for Evaluating Natural Finish)

Each test sample (0.5 g) (each of hair cosmetics prepared according to examples and comparative examples) was applied to a strand of virgin black hair having a length of 20 cm and a weight of 2 g, and the hair strand was combed and dried at room temperature. The dried hair strand was subjected to a sensory test by ten women special panelists, in which whether the hair strand had natural finish was sensorially scored according to the following scoring criteria, and the natural finish of the sample was evaluated according to the following evaluation criteria. In the scoring criteria, a higher score means a more satisfactorily natural finish.

(Scoring Criteria)

Score 5: Very natural finish
Score 4: Natural finish
Score 3: Slightly stiff finish
Score 2: Somewhat stiff finish
Score 1: Very stiff finish

(Evaluation Criteria)

AAA: Total score of the ten women special panelists is 40 or more, and eight or more panelists evaluate as Score 5
AA: Total score of the ten women special panelists is 40 or more, and five or more panelists evaluate as Score 5
A: Total score of the ten women special panelists is 40 or more
B: Total score of the ten women special panelists is 30 or more and less than 40
C: Total score of the ten women special panelists is 20 or more and less than 30
D: Total score of the ten women special panelists is less than 20

(Method for Evaluating Touch)

Each test sample (0.5 g) (each of hair cosmetics prepared according to examples and comparative examples) was applied to a strand of virgin black hair having a length of 20 cm and a weight of 4 g, and the hair strand was combed and dried at room temperature. The dried hair strand was subjected to a sensory test by ten women special panelists, in which the touch (pliableness and smoothness) of the hair strand was sensorially scored according to the following scoring criteria, and the touch of the sample was evaluated according to the following evaluation criteria. In the scoring criteria, a higher score means a more satisfactorily tough.

(Scoring Criteria)

Score 5: Very good touch
Score 4: Good touch
Score 3: Slightly poor touch
Score 2: Somewhat poor touch
Score 1: Very poor touch

(Evaluation Criteria)

AAA: Total score of the ten women special panelists is 40 or more, and eight or more panelists evaluate as Score 5
AA: Total score of the ten women special panelists is 40 or more, and five or more panelists evaluate as Score 5
A: Total score of the ten women special panelists is 40 or more
B: Total score of the ten women special panelists is 30 or more and less than 40
C: Total score of the ten women special panelists is 20 or more and less than 30
D: Total score of the ten women special panelists is less than 20

(Method for Evaluating Flaking)

Each test sample (0.5 g) (each of hair cosmetics prepared according to examples and comparative examples) was applied to a strand of virgin black hair having a length of 20 cm and a weight of 2 g, and the hair strand was combed and dried at room temperature. The dried hair strand was subjected to a sensory test by ten women special panelists, in which to which extent flaking occurred upon combing was sensorially scored according to the following scoring criteria, and the flaking of the sample was evaluated according to the following evaluation criteria. In the scoring criteria, a higher score means that less faking occurs.

(Scoring Criteria)

Score 5: No or substantially no flaking occurs
Score 4: Little flaking occurs
Score 3: Slight flaking occurs
Score 2: Somewhat flaking occurs
Score 1: Significant flaking occurs

(Evaluation Criteria)

AAA: Total score of the ten women special panelists is 40 or more, and eight or more panelists evaluate as Score 5
AA: Total score of the ten women special panelists is 40 or more, and five or more panelists evaluate as Score 5
A: Total score of the ten women special panelists is 40 or more
B: Total score of the ten women special panelists is 30 or more and less than 40
C: Total score of the ten women special panelists is 20 or more and less than 30
D: Total score of the ten women special panelists is less than 20

(Method for Evaluating Hair Style Retentivity)

Each test sample (0.5 g) (each of hair cosmetics prepared according to examples and comparative examples) was applied to virgin black hair having a length of 20 cm and a weight of 2 g, and the applied hair was immediately curled with a curler having a curling diameter of 2 cm, and the curled hair was dried at 50°C for one hour. The length of the curled hair strand was measured, and this length was defined as an initial length (L0).
Next, the dried hair strand was hanged from a board with scales, placed in a thermo-hygrostat at a temperature of 30°C

and relative humidity of 90% for three hours, then the length of the hair strand was measured, and this length was defined as a length after humidification (L2).
The length of the hair strand is the maximum diameter of the curl when the hair strand remains being curled, and it is the maximum length from an end of the hair near to the hair root (e.g., the length between an end near to hair root and another end near to hair tip) when the curled hair is partially or fully uncurled.
Next, a curling-retention rate of each sample was calculated according to the following equation, and the resistance to excess moisture was evaluated according to the following evaluation criteria:

$$\text{Curling-retention rate (\%)} = \{(20-L2)/(20-L0)\} \times 100$$

A sample has a higher curling retentivity and higher resistance to excess moisture (i.e., more superior hair style retentivity) when it has a higher curling-retention rate approaching 100%.

(Evaluation Criteria)

AAA: Curling-retention rate is 98% or more
AA: Curling-retention rate is 95% or more
A: Curling-retention rate is 90% or more
B: Curling-retention rate is 70% or more and less than 90%
C: Curling-retention rate is 50% or more and less than 70%
D: Curling-retention rate is less than 50%

(Method for Evaluating Washability)

The virgin black hair after the evaluation of touch (pliableness and smoothness) according to the "method for evaluating touch" was subjected to a washing test by ten women special panelists, in which the washability was sensorially scored according to the following scoring criteria, and the washability of each sample was evaluated according to the following evaluation criteria. In the scoring criteria, a sample with a higher score can be more satisfactorily washed out from the hair.

(Scoring Criteria)

Score 5: Very good washability
Score 4: Good washability
Score 3: Slightly poor washability
Score 2: Somewhat poor washability
Score 1: Very poor washability

(Evaluation Criteria)

AAA: Total score of the ten women special panelists is 40 or more, and eight or more panelists evaluate as Score 5
AA: Total score of the ten women special panelists is 40 or more, and five or more panelists evaluate as Score 5
A: Total score of the ten women special panelists is 40 or more
B: Total score of the ten women special panelists is 30 or more and less than 40
C: Total score of the ten women special panelists is 20 or more and less than 30
D: Total score of the ten women special panelists is less than 20

(Preparation Example 1 of Amino-Containing Alkoxysilanes)

A 1:1 (by mole) mixture of 3-aminopropyltriethoxysilane and lauryl acrylate was prepared, followed by a reaction

at 50°C for seven days, to give an amino-containing alkoxysilane as a reaction product.

(Preparation Example 2 of Amino-Containing Alkoxysilanes)

A 1:1 (by mole) mixture of γ-aminopropyltriethoxysilane (trade name "KBE-903", supplied by Shin-Etsu Chemical Co., Ltd.) and lauryl acrylate was prepared in an atmosphere of nitrogen gas, followed by a reaction at 40°C for ten days, to give an amino-containing alkoxysilane as a reaction product.

(Preparation Example 1 of Urethane Polymer Dispersions)

In a four-necked separable flask equipped with a stirrer, a nitrogen inlet tube, a thermometer, and a condenser were placed 150 parts of a product under the trade name "NS 2400" (supplied by ADEKA CORPORATION, a polyester diol, number-average molecular weight: 2000, hydroxyl value: 56.1 mg-KOH/g), 15 parts of 2,2-dimethylolbutanoic acid (hydroxyl value: 754.0 mg-KOH/g), 8 parts of 1,4-butanediol, 66.4 parts of isophorone diisocyanate [isocyanate content (NCO content): 37.8%; IPDI], 180 parts of methyl methacrylate, and 100 parts of butyl acrylate, followed by a reaction at temperatures of from 75°C to 80°C under nitrogen gas flow for three hours, to yield a reaction mixture containing a carboxyl-containing and terminal-isocyanate-containing polymer, having a residual isocyanate content of 1.2%.

Next, the whole quantity of the reaction mixture containing the carboxyl-containing and terminal-isocyanate-containing polymer was combined with 31.6 parts of the amino-containing alkoxysilane prepared according to "Preparation Example 1 of amino-containing alkoxysilanes", followed by a reaction at temperatures of from 75°C to 80°C under nitrogen gas flow for one hour, to yield a reaction mixture containing a carboxyl-containing alkoxysililated urethane polymer.

This carboxyl-containing alkoxysililated urethane polymer was cooled to 40°C and combined with 1310 parts of deionized water containing 7.5 parts of triethanolamine and 2 parts of sodium hydroxide with high-speed stirring, to yield an aqueous dispersion containing 15 percent by mass of a hydrolyzable sililated urethane polymer and 15 percent by mass of an acrylic monomer (monomer-containing sililated urethane polymer aqueous dispersion).

(Preparation Example 2 of Urethane Polymer Dispersions)

In a four-necked separable flask equipped with a stirrer, a nitrogen inlet tube, a thermometer, and a condenser, were placed 100 parts of a product under the trade name "PTMG 2000" (supplied by Mitsubishi Chemical Corporation, a poly(tetramethylene glycol), number-average molecular weight: 2000, hydroxyl value: 57.4 mg-KOH/g), 13.34 parts of 2,2-dimethylolbutanoic acid (hydroxyl value: 754.0 mg-KOH/g), 6 parts of 1,4-butanediol (hydroxyl value: 90.1 mg-KOH/g), 55.41 parts of isophorone diisocyanate [isocyanate content (NCO content): 37.8%; IPDI], 0.02 part of dibutyltin dilaurate, and 100 parts of methyl ethyl ketone, followed by a reaction at temperatures of from 80°C to 85°C under nitrogen gas flow for six hours, to yield a reaction mixture containing a carboxyl-containing and terminal-isocyanate-containing polymer having a residual isocyanate content of 2.0%.

Next, the whole quantity of the reaction mixture containing the carboxyl-containing and terminal-isocyanate-containing polymer was combined with 19.22 parts of the amino-containing alkoxysilane prepared according to "Preparation Example 2 of Amino-Containing Alkoxysilanes", followed by a reaction at temperatures of from 80°C to 85°C under nitrogen gas flow for one hour reaction, to give a reaction mixture containing a carboxyl-containing alkoxysililated urethane polymer.

The carboxyl-containing alkoxysililated urethane polymer was cooled to 40°C, with high-speed stirring, combined with a solution which had been prepared by mixing and dissolving 6.72 parts of triethanolamine, 1.80 parts of sodium hydroxide, and 2.83 parts of isophoronediamine in 500 parts of deionized water and methyl ethyl ketone was distilled off therefrom at temperatures of from 40°C to 45°C under reduced pressure.

Next, a proper amount of ion-exchanged water was added, to yield an aqueous dispersion containing 40 percent by mass of a urethane resin (sililated urethane polymer aqueous dispersion).

(Preparation Example 1 of Urethane-Modified Acrylic Polymers)

A monomer emulsion was prepared by weighing 80 parts of 2-ethylhexyl acrylate (2EHA), 90 parts of butyl methacrylate (BMA), 150 parts of cyclohexyl methacrylate (CHMA), and 20 parts of γ-methacryloxypropylmethyldiethoxysilane (trade name "KBE-502" supplied by Shin-Etsu Chemical Co., Ltd.) respectively, and emulsifying these monomers in 240 parts of deionized water with 10 parts of an emulsifier under the trade name "ADEKA REASOAP SR-10" [supplied by ADEKA CORPORATION].

Next, in a four-necked separable flask equipped with a stirrer, a nitrogen inlet tube, a thermometer, and a reflux condenser were placed 450 parts of the monomer-containing sililated urethane polymer aqueous dispersion prepared

according to "Preparation Example 1 of Urethane Polymer Dispersions" and 180 parts of deionized water, and the inner temperature of the separable flask was raised to 80°C. After the temperature rise, the monomer emulsion and 2 parts of potassium persulfate (KPS) as a polymerization initiator were continuously uniformly added dropwise into the separable flask separately through different inlets over two hours and thereby yielded an aqueous dispersion containing a urethane-modified acrylic polymer and having non-volatile content (solids content) of 40 percent by mass (hereinafter also referred to as a "urethane-modified acrylic polymer aqueous dispersion A"). The urethane-modified acrylic polymer in the urethane-modified acrylic polymer aqueous dispersion A is a urethane-modified acrylic binary copolymer.

(Preparation Example 2 of Urethane-Modified Acrylic Polymers)

A monomer emulsion was prepared by weighing 80 parts of 2-ethylhexyl acrylate (2EHA), 90 parts of butyl methacrylate (BMA), 150 parts of cyclohexyl methacrylate (CHMA), and 30 parts of γ-methacryloxypropylmethyldiethoxysilane (trade name "KBE-502" supplied by Shin-Etsu Chemical Co., Ltd.) respectively, and emulsifying these monomers in 240 parts of deionized water with 10 parts of an emulsifier under the trade name "ADEKA REASOAP SR-10" [supplied by ADEKA CORPORATION].

Next, in a four-necked separable flask equipped with a stirrer, a nitrogen inlet tube, a thermometer, and a reflux condenser were placed 450 parts of the monomer-containing sililated urethane polymer aqueous dispersion prepared according to "Preparation Example 1 of Urethane Polymer Dispersions", 220 parts of deionized water, and 30 parts of γ-methacryloxypropylmethyldiethoxysilane (trade name "KBE-502" supplied by Shin-Etsu Chemical Co., Ltd.). The temperature of the mixture was raised to 40°C with stirring in an atmosphere of nitrogen gas, reacted for one hour while maintaining the temperature (40°C), and thereby yielded a silicone-urethane copolymer.

After the inner temperature of the separable flask was raised to 80°C, the above-prepared monomer emulsion and 2 parts of potassium persulfate (KPS) as a polymerization initiator were continuously uniformly added dropwise into the separable flask separately through different inlets over two hours and thereby yielded an aqueous dispersion containing a urethane-modified acrylic polymer and having a non-volatile content of 40 percent by mass (hereinafter also referred to as a "urethane-modified acrylic polymer aqueous dispersion B"). The urethane-modified acrylic polymer in the urethane-modified acrylic polymer aqueous dispersion B is a urethane-modified acrylic ternary copolymer.

(Preparation Example 3 of Urethane-Modified Acrylic Polymers)

A monomer emulsion was prepared by weighing 80 parts of 2-ethylhexyl acrylate (2EHA), 90 parts of butyl methacrylate (BMA), 150 parts of cyclohexyl methacrylate (CHMA), 15 parts of γ-methacryloxypropylmethyldiethoxysilane (trade name "KBE-502" supplied by Shin-Etsu Chemical Co., Ltd.), and 15 parts of γ-methacryloxypropyltriethoxysilane (trade name "KBE-503" supplied by Shin-Etsu Chemical Co., Ltd.) respectively, and emulsifying these monomers in 240 parts of deionized water with 10 parts of an emulsifier under the trade name "ADEKA REASOAP SR-10" [supplied by ADEKA CORPORATION].

Next, in a four-necked separable flask equipped with a stirrer, a nitrogen inlet tube, a thermometer, and a reflux condenser were placed 450 parts of the monomer-containing sililated urethane polymer aqueous dispersion prepared according to "Preparation Example 1 of Urethane Polymer Dispersions", 220 parts of deionized water, 15 parts of γ-methacryloxypropylmethyldiethoxysilane (trade name "KBE-502" supplied by Shin-Etsu Chemical Co., Ltd.), and 15 parts of γ-methacryloxypropyltriethoxysilane (trade name "KBE-503" supplied by Shin-Etsu Chemical Co., Ltd.). The temperature of the mixture was raised to 40°C with stirring in an atmosphere of nitrogen gas, reacted for one hour while maintaining the temperature (40°C), and thereby yielded a silicone-urethane copolymer.

After the inner temperature of the separable flask was raised to 80°C, the above-prepared monomer emulsion and 2 parts of potassium persulfate (KPS) as a polymerization initiator were continuously uniformly added dropwise into the separable flask separately through different inlets over two hours, to yield an aqueous dispersion containing a urethane-modified acrylic polymer and having a non-volatile content of 40 percent by mass (hereinafter also referred to as a "urethane-modified acrylic polymer aqueous dispersion C"). The urethane-modified acrylic polymer in the urethane-modified acrylic polymer aqueous dispersion C is a urethane-modified acrylic ternary copolymer.

(Preparation Example 4 of Urethane-Modified Acrylic Polymers)

A monomer emulsion was prepared by weighing 80 parts of 2-ethylhexyl acrylate (2EHA), 90 parts of butyl methacrylate (BMA), 150 parts of cyclohexyl methacrylate (CHMA), 20 parts of γ-methacryloxypropylmethyldiethoxysilane (trade name "KBE-502" supplied by Shin-Etsu Chemical Co., Ltd.), and 20 parts of a silicone alkoxy oligomer (trade name "X-40-9238" supplied by Shin-Etsu Chemical Co., Ltd.) respectively, and emulsifying these monomers in 240 parts of deionized water with 10 parts of an emulsifier under the trade name "ADEKA REASOAP SR-10" (supplied by ADEKA CORPORATION).

Next, in a four-necked separable flask equipped with a stirrer, a nitrogen inlet tube, a thermometer, and a reflux condenser were placed 450 parts of the monomer-containing sililated urethane polymer aqueous dispersion prepared according to "Preparation Example 1 of Urethane Polymer Dispersions", 220 parts of deionized water, and 20 parts of γ-methacryloxypropylmethyldiethoxysilane (trade name "KBE-502" supplied by Shin-Etsu Chemical Co., Ltd.). The temperature of the mixture was raised to 40°C with stirring in an atmosphere of nitrogen gas, reacted for one hour while maintaining the temperature (40°C), and thereby yielded a silicone-urethane copolymer.

After the inner temperature of the separable flask was raised to 80°C, the above-prepared monomer emulsion and 2 parts of potassium persulfate (KPS) as a polymerization initiator were continuously uniformly added dropwise into the separable flask separately through different inlets over two hours, to yield an aqueous dispersion containing a urethane-modified acrylic polymer and having a non-volatile content of 40 percent by mass (hereinafter also referred to as a "urethane-modified acrylic polymer aqueous dispersion D"). The urethane-modified acrylic polymer in the urethane-modified acrylic polymer aqueous dispersion D is a urethane-modified acrylic ternary copolymer.

(Preparation Example 5 of Urethane-Modified Acrylic Polymers)

A monomer emulsion was prepared by weighing 60 parts of methyl methacrylate (MMA) and 5 parts of γ-methacryloxypropyltriethoxysilane (trade name "KBE-503" supplied by Shin-Etsu Chemical Co., Ltd.) respectively, and emulsifying these monomers in 120 parts of deionized water with 2 parts of an emulsifier under the trade name "ADEKA REASOAP SR-10" (supplied by ADEKA CORPORATION).

Next, in a four-necked separable flask equipped with a stirrer, a nitrogen inlet tube, a thermometer, and a reflux condenser were placed 750 parts of the sililated urethane polymer aqueous dispersion prepared according to "Preparation Example 2 of Urethane Polymer Dispersions", 300 parts of deionized water, and 5 parts of γ-methacryloxypropyltriethoxysilane (trade name "KBE-503" supplied by Shin-Etsu Chemical Co., Ltd.). The temperature of the mixture was raised to 40°C with stirring in an atmosphere of nitrogen gas, reacted for one hour while maintaining the temperature (40°C), and thereby yielded a silicone-urethane copolymer.

After the inner temperature of the separable flask was raised to 80°C, the above-prepared monomer emulsion and 2 parts of potassium persulfate (KPS) as a polymerization initiator were continuously uniformly added dropwise into the separable flask separately through different inlets over two hours, and thereby yielded an aqueous dispersion containing a urethane-modified acrylic polymer and having a non-volatile content of 30 percent by mass (hereinafter also referred to as a "urethane-modified acrylic polymer aqueous dispersion E"). The urethane-modified acrylic polymer in the urethane-modified acrylic polymer aqueous dispersion E is a urethane-modified acrylic ternary copolymer.

(EXAMPLE 1)

A solution was prepared by mixing 10.0 parts of a liquid paraffin (trade name "HIWHITE 22S" supplied by Nippon Oil Corporation), 10.0 parts of a microcrystalline wax (trade name "Microcrystalline Wax-P" supplied by Nikko Rica Corporation), 4.0 parts of dimethylpolysiloxane (trade name "KF-96A-6cs" supplied by Shin-Etsu Chemical Co., Ltd.; viscosity (25C): 6 mPa.s), 4.0 parts of stearyl alcohol, 3.0 parts of carnauba wax (trade name "Carnauba Wax" supplied by CERARICA NODA Co., Ltd.), 0.5 part of isostearic acid, 4.5 parts of stearic acid, 2.0 parts of pentaerythritol tetra-2-ethylhexanoate, 3.0 parts of a polyoxyethylene hydrogenated caster oil (average number of moles of ethylene oxide repeating units in polyoxyethylene moiety: 60) (trade name "EMALEX HC-60" supplied by Nihon Emulsion Co., Ltd.), 2.0 parts of a polyoxyethylene oleyl ether phosphate (average number of moles of ethylene oxide repeating units in polyoxyethylene moiety: 10) (trade name "Crodafos N10A" supplied by Croda Japan K.K.), 3.0 parts of self-emulsifiable glyceryl monostearate, and a proper amount of a perfume; and heating the mixture at 80°C with stirring. Independently, a mixture was prepared by mixing 33 parts of ion-exchanged water and 10.0 parts of propylene glycol. The mixture was heated to 80°C, combined with and dissolved in the above-prepared solution. The resulting mixture was further combined with 1.0 part of triethanolamine and emulsified with a homomixer. While maintaining at 80°C with stirring, the resulting emulsion was further sequentially combined with a proper amount of sodium polyacrylate (trade name "ARONVIS S" supplied by Nihon Junyaku Co., Ltd.), a proper amount of p-hydroxybenzoic ester (trade name "Mekkins M" supplied by Ueno Fine Chemicals Industry, Ltd.), 10.0 parts of the urethane-modified acrylic polymer aqueous dispersion A, prepared according to Preparation Example 1 of urethane-modified acrylic polymers (4.0 parts in terms of solids content of urethane-modified acrylic polymer), and the mixture was rapidly cooled to 35°C, to yield a wax-type hair cosmetic (hair wax).

(EXAMPLE 2)

A hair cosmetic stock was prepared and a wax-type hair cosmetic was produced by the procedure as described in Example 1, except for using, instead of the urethane-modified acrylic polymer aqueous dispersion A, the urethane-modified acrylic polymer aqueous dispersion B, prepared according to Preparation Example 2 of urethane-modified acrylic polymers, in such a proportion that the amount of the urethane-modified acrylic polymer B is 4 percent by mass based on the total amount of the wax-type hair cosmetic.

(EXAMPLE 3)

A hair cosmetic stock was prepared and a wax-type hair cosmetic was produced by the procedure as described in Example 1, except for using, instead of the urethane-modified acrylic polymer aqueous dispersion A, the urethane-modified acrylic polymer aqueous dispersion C, prepared according to Preparation Example 3 of urethane-modified acrylic polymer, in such a proportion that the amount of the urethane-modified acrylic polymer C is 4 percent by mass based on the total amount of the wax-type hair cosmetic.

(EXAMPLE 4)

A hair cosmetic stock was prepared and a wax-type hair cosmetic was produced by the procedure as described in Example 1, except for using, instead of the urethane-modified acrylic polymer aqueous dispersion A, the urethane-modified acrylic polymer aqueous dispersion D, prepared according to Preparation Example 4 of urethane-modified acrylic polymers, in such a proportion that the amount of the urethane-modified acrylic polymer D is 4 percent by mass based on the total amount of the wax-type hair cosmetic.

(EXAMPLE 23)

A hair cosmetic stock was prepared and a wax-type hair cosmetic was produced by the procedure as described in Example 1, except for using, instead of the urethane-modified acrylic polymer aqueous dispersion A, the urethane-modified acrylic polymer aqueous dispersion E, prepared according to Preparation Example 5 of urethane-modified acrylic polymers, in such a proportion that the amount of the urethane-modified acrylic polymer E is 4 percent by mass based on the total amount of the wax-type hair cosmetic.

(COMPARATIVE EXAMPLE 1)

A wax-type hair cosmetic was prepared by the procedure as described in Example 1, except for using, instead of the urethane-modified acrylic polymer aqueous dispersion A, the copolymer 11, described in Japanese Patent No. 3670841, in such a proportion that the amount of the copolymer 11 is 4 percent by mass based on the total amount of the wax-type hair cosmetic.

The copolymer 11 described in Japanese Patent No. 3670841 can be prepared by dissolving, as monomer components, 50 percent by mass of 3-methacryloxypropyltrimethoxysilane, 40 percent by mass of methyl methacrylate, and 10 percent by mass of "CH3-C(=CH2)-C(=O)-O-(CH2)3-Si(CH3)(CH3)-[O-Si(CH3)(CH3)]n-O-Si(CH3)(CH3)-(CH2) 3-C(=)-C=CH2)-CH3" (weight-average molecular weight: 5000) in 100 ml of ethanol, heating and stirring the mixture at 70°C under nitrogen gas flow for one hour, adding 0.05 g of potassium persulfate, carrying out a reaction overnight to complete a copolymerization reaction, cooling the reaction mixture to room temperature, concentrating the mixture under reduced pressure, dissolving the residue in 10 ml of ethanol, placing the resulting solution into 500 ml of n-hexane to give precipitates, and fractionating the precipitates.

(COMPARATIVE EXAMPLE 2)

A wax-type hair cosmetic was prepared by the procedure as described in Example 1, except for using, instead of the urethane-modified acrylic polymer aqueous dispersion A, a sililated urethane polymer aqueous dispersion prepared by the following method for preparing a sililated urethane polymer aqueous dispersion, in such a proportion that the amount of the sililated urethane polymer in the sililated urethane polymer aqueous dispersion is 4 percent by mass based on the total amount of the wax-type hair cosmetic.

(Method for Preparing Sililated Urethane Polymer Aqueous Dispersion)

Initially, an amino-containing alkoxysilane was prepared by mixing 221.4 g of γ-aminopropyltriethoxysilane (trade name "KBE-903" supplied by Shin-Etsu Chemical Co., Ltd.) and 240.4 g of lauryl acrylate in an atmosphere of nitrogen gas, and reacting the components at 40°C for ten days.

Next, in a four-necked flask equipped with a nitrogen inlet tube, a thermometer, a condenser, and a stirrer were placed 100.00 parts of a poly(tetramethylene glycol) (trade name "PTMG 2000" supplied by Mitsubishi Chemical Corporation; number-average molecular weight: 2000, hydroxyl value: 57.4 mg-KOH/g), 6.00 parts of 1,4-butanediol (hydroxyl value:90.1 mg-KOH/g), 13.34 parts of 2,2-dimethylolbutanoic acid (hydroxyl value: 754.0 mg-KOH/g), 55.41 parts of isophorone diisocyanate (isocyanate content: 37.8%), 0.02 part of dibutyltin dilaurate, and 100 parts of methyl ethyl ketone. The mixture was subjected to a reaction at temperatures of from 80°C to 85°C under nitrogen gas flow for six hours and thereby yielded a reaction mixture containing a urethane prepolymer having a content of residual isocyanate of 2.0%. The reaction mixture was combined with the above-prepared amino-containing alkoxysilane, followed by a reaction at temperatures of from 80°C to 85°C under nitrogen gas flow for one hour. The reaction mixture was cooled to 40°C, combined with an aqueous solution which had been prepared by dissolving 1.80 parts of sodium hydroxide, 6.72 parts of triethanolamine, and 2.83 parts of isophoronediamine in 500.00 parts of ion-exchanged water with high-speed stirring, and methyl ethyl ketone was distilled off from the mixture at 40°C to 45°C under reduced pressure. The residue was combined with a proper amount of ion-exchanged water and thereby yielded the sililated urethane polymer aqueous dispersion having a urethane resin concentration of 40 percent by mass.

(COMPARATIVE EXAMPLE 3)

A wax-type hair cosmetic was prepared by the procedure as described in Example 1, except for using, instead of the urethane-modified acrylic polymer aqueous dispersion A, a mixture of 50 parts by mass of the hair styling resin A prepared according to Material Preparation Example 1 of JP-A No. Hei 8-92044 and 50 parts by mass of the cosmetic composition prepared according to Example 1 of JP-A No. 2003-171236, in such a proportion that the resin content of the mixture is 4 percent by mass based on the total amount of the wax-type hair cosmetic.

The hair styling resin A prepared according to Material Preparation Example 1 of JP-A No. Hei 8-92044 can be prepared in the following manner. Specifically, in a 1-liter four-necked flask equipped with a reflux condenser, a thermometer, a glass tube for nitrogen replacement, a dropping funnel, and a stirrer were placed 200 parts of a polymerizable monomer mixture, 100 parts of ethyl alcohol, and 2 parts of a polymerization initiator. The polymerizable monomer mixture contained 20 parts of acrylic acid, 35 parts of lauryl methacrylate, 35 parts of butyl methacrylate, and 10 parts of ethyl methacrylate. The resulting mixture in the flask was subjected to polymerization under reflux (at about 80°C) under nitrogen gas flow for five hours, and the reaction mixture after polymerization was cooled and combined with a solution of a water-soluble organic basic substance [aminomethylpropanol (AMP)] in ethyl alcohol at 50°C.

The cosmetic composition prepared according to Example 1 of JP-A No. 2003-171236 can be prepared in the following manner. Specifically, in glass four-necked flask equipped with a stirrer, a thermometer, a nitrogen inlet tube, and a reflux condenser, were placed 70 g of isophorone diisocyanate (IPDI), 220 g of a polyesterpolyol (1,6-hexanediol adipate, number-average molecular weight: 2,000), 8 g of a compound represented by "C2H5-C(CH2OH)(CH2OH)-CH2-O-C3H6-Si(CH3)(CH3)-[O-Si(CH3)(CH3)]m-CH3" (having hydroxyl group at one of two terminals, number-average molecular weight: about 1,000), and 14 g of dimethylolbutanoic acid (DMBA). The mixture was further combined with 50 g of ethyl acetate as a solvent, reacted by heating on an oil bath at 80°C for four hours, further combined with additional portions of 2 g of N-methyldiethanolamine (NMDEtA) and 60 g of ethyl acetate, and further reacted at 80°C for two hours, to yield a prepolymer containing residual isocyanato groups (NCO groups). The prepolymer containing residual NCO groups is cooled to 50°C, dispersed in 900 g of water containing 9 g of triethylamine with high-speed stirring, subjected to a chain-extension reaction at 50°C for three hours to have a higher molecular weight. The ethyl acetate was recovered from the resulting aqueous mixture to yield an aqueous composition of an amphoteric urethane resin containing polysiloxane chains but containing substantially no solvent to thereby give the cosmetic composition.

The wax-type hair cosmetics (hair waxes) prepared according to Examples 1 to 4 and 23, and those prepared according to Comparative Examples 1 to 3 were evaluated according to the above evaluation methods on "less sticky feeling during a duration after application and before drying", "hair styling capability", "natural finish", "touch", "flaking", "hair style retentivity", and "washability". The evaluation results are shown in Table 1. Data on the "less sticky feeling during a duration after application and before drying" are shown in "sticky feeling before drying" in Table 1. Data on the other items are shown in respective fields.

[Table 1]

TABLE 1

| | Examples | | | | | Comparative Examples | | |
|---|---|---|---|---|---|---|---|---|
| | 1 | 2 | 3 | 4 | 23 | 1 | 2 | 3 |
| Sticky feeling before drying | A | AA | AA | AAA | AA | B | A | D |
| Hair styling capability | AA | A | AA | A | AAA | B | A | A |
| Natural finish | A | AA | A | AAA | A | C | B | D |

(continued)

| | Examples | | | | | Comparative Examples | | |
|---|---|---|---|---|---|---|---|---|
| | 1 | 2 | 3 | 4 | 23 | 1 | 2 | 3 |
| Touch | A | AA | AA | AAA | A | B | B | D |
| Flaking | A | A | A | A | A | A | C | C |
| Hair style retentivity | A | AA | AA | AAA | AAA | B | B | C |
| Washability | A | A | A | A | A | D | A | A |

Table 1 demonstrates that the hair cosmetics according to Examples 1 to 4 and 23, which use urethane-modified acrylic polymers as film-formable polymer components, exhibit hair styling capability, natural finishing capability, and hair style retentivity respectively at superior levels; and that among these hair cosmetics, those according to Examples 2 to 4, which use acrylic-rich urethane-modified acrylic ternary copolymers as film-formable polymer components, exhibit natural finishing capability and smooth touch at furthermore superior levels to show good touch at furthermore superior levels. Table 1 also demonstrates that the hair cosmetic according to Example 23, which uses a urethane-rich urethane-modified acrylic ternary copolymer as a film-formable polymer component, exhibits hair styling capability and hair style retentivity at furthermore superior levels.

Specifically, the hair cosmetic according to Example 1 has a high hair styling capability, can set the hair with a natural finish, and can exhibit high resistance to excess moisture to prevent the set hair style from unsetting even at high humidity. The hair cosmetic according to Example 2 shows less sticky feeling upon application to the hair, imparts a more satisfactory touch to the set hair, imparts a furthermore natural finish to the set hair, and can retain the set hair style furthermore satisfactorily. The hair cosmetic according to Example 3 shows less sticky feeling upon application to the hair, imparts a more satisfactory touch to the set hair, enables hair styling more satisfactorily, and can retain the set hair style further satisfactorily even at high humidity. The hair cosmetic according to Example 4 shows further less sticky feeling upon application to the hair, imparts a furthermore satisfactory touch to the set hair, imparts a furthermore natural finish to the set hair, can retain the set hair style furthermore satisfactorily even at high humidity, gives a good touch, helps the fingers to pass through the hair, helps the set curled hair to be resistant to uncurling even when the set hair is combed by fingers, and enables the set hair style to be in a natural finish. On the other hand, the hair cosmetic according to Example 23 has further higher hair styling capability and can retain the set hair style furthermore satisfactorily, even though it shows a satisfactory touch and a high easiness for passage of fingers.

The results therefore demonstrate that the hair cosmetics according to these examples enable the hair to set without any sticky feeling during a duration from the application to drying, enable the hair to set to a predetermined hair style highly satisfactorily, impart a natural feeling and a pliable and smooth touch to the set hair without causing flaking even upon combing, and can retain the hair style, such as curled hair, even at high humidity. Additionally, they can be washed out from the hair with superior washability.

In contrast, the hair cosmetic according to Comparative Example 1 contains a film-formable polymer component of an acrylic resin having a hydrolyzable silicon group, causes sticky feeling upon drying, gives coarse/stiff feeling to the set hair without natural touch, and is washed out from the hair insufficiently. The hair cosmetic according to Comparative Example 2 contains a film-formable polymer component of a urethane resin having a hydrolyzable silicon group, causes flaking, gives poor natural finish to the hair, and thereby exhibits insufficient functions. The hair cosmetic according to Comparative Example 3 contains a film-formable polymer component including an acrylic resin and a urethane resin separately without bonding through a linkage segment, gives a highly sticky feeling upon drying, gives a highly coarse/stiff feeling to the set hair with poor touch, causes flaking, and exhibits significantly insufficient functions.

(EXAMPLE 5)

Initially, an emulsion component was prepared by adding 2.5 parts of glycerol, 2.5 parts of 1,3-butylene glycol, and 0.5 part of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.) to 5 parts of ion-exchanged water at room temperature; further adding 1.0 part of dimethylpolysiloxane [trade name "SH200 C Fluid 1000cs" supplied by Dow Corning Toray Co., Ltd.; viscosity (25°C): 1,000 mPa.s]; emulsifying the mixture with a homomixer, and further adding 10 parts of ion-exchanged water thereto.

Independently, a solution was prepared by homogenously dissolving 0.7 part of a carboxyvinyl polymer (trade name "HIVISWAKO 105" supplied by Wako Pure Chemical Industries, Ltd.), 5.0 parts (2.0 parts in terms of solids content of urethane-modified acrylic polymer) of the urethane-modified acrylic polymer aqueous dispersion D prepared according

to Preparation Example 4 of urethane-modified acrylic polymers, a proper amount of a 10% aqueous sodium hydroxide solution so as to have a pH of 7.5, 20.0 parts of ethanol, 0.1 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.1 part of a perfume, 0.03 part of trisodium edetate, and 5.0 parts of a product under the trade name "Yukaformer 301" (supplied by Mitsubishi Chemical Corporation; a N-methacryloyloxyethyl-N,N-dimethylammonium-$\alpha$-N-methylcarboxybetaine-alkyl methacrylate copolymer solution; content of active ingredient: 30 percent by mass) in 47.57 parts of ion-exchanged water. The prepared solution was combined with the above-prepared emulsion component and thereby yielded a gel-type hair cosmetic (emulsion-type hair styling gel).

(EXAMPLE 6)

Initially, an emulsion component was prepared by adding 2.5 parts of glycerol, 1.0 part of urea, and 0.5 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.) to 5 parts of ion-exchanged water at room temperature; further adding 1.0 part of a highly polymerized amino-modified dimethylpolysiloxane {the amino-modified high-molecular-weight silicone "(CH3)3-Si-O-(Si(CH3)(CH3)-O]5000-[Si(CH3)((CH2)3N(CH3)2)-O]5-Si-(CH3)3" described in Example 1 of JP-A No. Hei 5-85918}; emulsifying the mixture with a homomixer; and further adding 10 parts of ion-exchanged water.

Independently, a solution was prepared by homogeneously dissolving 0.7 part of an acrylic acid/alkyl methacrylate copolymer (trade name "PEMULEN TR-1" supplied by B.F. Goodrich Chemical Company), 12.0 parts (4.8 parts in terms of solids content of urethane-modified acrylic polymer) of the urethane-modified acrylic polymer aqueous dispersion C prepared according to Preparation Example 3 of urethane-modified acrylic polymers, a proper amount of a 10% aqueous sodium hydroxide solution so as to have a pH of 7.5, 20.0 parts of ethanol, 0.1 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.1 part of a perfume, 0.03 part of trisodium edetate, and 5.0 parts of a polyether-modified dimethylpolysiloxane (trade name "SILWET 10-E" supplied by Nippon Unicar Co., Ltd.) in 59.07 parts of ion-exchanged water. The solution was combined with the above-prepared emulsion component and thereby yielded a gel-type hair cosmetic (emulsion-type hair styling gel).

(EXAMPLE 7)

Initially, an emulsion component was prepared by adding 2.5 parts of diglycerol, 2.5 parts of sorbitol, and 0.5 part of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.) to 5 parts of ion-exchanged water at room temperature; further adding 1.0 part of a methylphenylpolysiloxane (trade name "KF-56" supplied by Shin-Etsu Chemical Co., Ltd.); emulsifying the mixture with a homomixer; and further adding 10 parts of ion-exchanged water.

Independently, a solution was prepared by homogenously dissolving 0.7 part of a hydroxyethylcellulose (trade name "Natrosol 250LR" supplied by Hercules Incorporated, 10.0 parts (4.0 parts in terms of solids content of urethane-modified acrylic polymer) of the urethane-modified acrylic polymer aqueous dispersion B prepared according to Preparation Example 2 of urethane-modified acrylic polymers, a proper amount of a 10% aqueous sodium hydroxide solution so as to have a pH of 7.5, 20.0 parts of ethanol, 0.1 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.1 part of a perfume, 0.03 part of trisodium edetate, and 2.0 parts of a vinyl acetate/vinylpyrrolidone copolymer (trade name "PVP/VA S-630" supplied by General Aniline and Film Corporation) in 45.75 parts of ion-exchanged water. This solution was combined with the above-prepared emulsion component and thereby yielded a gel-type hair cosmetic (emulsion-type hair styling gel).

(EXAMPLE 8)

Initially, an emulsion component was prepared by adding 2.5 parts of maltitol, 2.5 parts of propylene glycol, and 0.5 part of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.) to 5 parts of ion-exchanged water at room temperature; further adding 1.0 part of an alkyl-modified dimethylpolysiloxane (trade name "KF-412" supplied by Shin-Etsu Chemical Co., Ltd.); emulsifying the mixture with a homomixer; and further adding 10 parts of ion-exchanged water.

Independently, a solution was prepared by homogenously dissolving 1.0 part of an alkyl acrylate/alkyl methacrylate/polyoxyethylene stearyl ether copolymer (trade name "ACULYN 22" supplied by ROHM AND HAAS Co.; average number of moles of ethylene oxide repeating units in polyoxyethylene moiety: 20), 2.5 parts (1.0 part in terms of solids

content of urethane-modified acrylic polymer) of the urethane-modified acrylic polymer aqueous dispersion A prepared according to Preparation Example 1 of urethane-modified acrylic polymers, a proper amount of a 10% aqueous sodium hydroxide solution so as to have a pH of 7.5, 20.0 parts of ethanol, 0.1 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.1 part of a perfume, and 5.0 parts of an ammonium-modified high-molecular-weight silicone {the amino-modified high-molecular-weight silicone "HO-Si(CH3)(CH3)-O-[Si(CH3)(CH3)-O]18000-[Si(CH3)((CH2)3N$^+$(CH3)3Cl$^-$)-O]2-Si(CH3)(CH3)-OH" described in Example 5 of JP-A No. Hei 5-85918} in 49.8 parts of ion-exchanged water. This solution was combined with the above-prepared emulsion component and thereby yielded a gel-type hair cosmetic (emulsion-type hair styling gel).

(EXAMPLE 9)

Initially, an emulsion component was prepared by adding 2.5 parts of glycerol, 2.5 parts of 1,3-butylene glycol, and 0.5 part of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.) to 5 parts of ion-exchanged water at room temperature; further adding 1.0 part of a dihydroxypolydimethylsiloxane [trade name "XF3802A" supplied by GE Toshiba Silicones Co., Ltd.; viscosity (25°C): 8x $10^4$ mPa.s]; emulsifying the mixture with a homomixer; and further adding 10 parts of ion-exchanged water.

Independently, a solution was prepared by homogenously dissolving 0.7 part of an associative thickener having urethane bonds {the associative thickener described in Example 12 of JP-A No. 2000-234085 [POE diisostearyl ether; average number of moles of ethylene oxide repeating units in polyoxyethylene (POE) moiety: 500, ratio by mass of hydrophilic moieties to hydrophobic moieties of 46:1]}, 12.0 parts (4.8 parts in terms of solids content of urethane-modified acrylic polymer) of the urethane-modified acrylic polymer aqueous dispersion D prepared according to Preparation Example 4 of urethane-modified acrylic polymers, 20.0 parts of ethanol, 0.1 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.1 part of a perfume, 0.03 part of trisodium edetate, and 5.0 parts of a high-molecular-weight polysiloxane (trade name "TSE 200A" supplied by GE Toshiba Silicones Co., Ltd.) in 40.57 parts of ion-exchanged water. This solution was combined with the above-prepared emulsion component and thereby yielded a gel-type hair cosmetic (emulsion-type hair styling gel).

The hair styling gels according to Examples 5 to 9 each have superior hair style retentivity and impart a superior touch to the set hair. Specifically, these hair styling gels, if used for hair styling of scalp hairs, enable hair styling of the scalp hairs with a natural feeling and a pliable and smooth touch and can retain the set hair style for a long time even in an atmosphere of high humidity.

(EXAMPLE 10)

Initially, a dispersion was prepared by dissolving, in 51 parts of ion-exchanged water, 8 parts of propylene glycol and 2 parts of diglycerol; further adding 0.5 part of an acrylic acid/alkyl methacrylate copolymer (trade name "PEMULEN TR-2" supplied by B.F. Goodrich Chemical Company); dispersing the mixture with a homomixer; heating the mixture to 80°C; adding 1 part of silica particles (average particle diameter: 5 μm, spherical shape), 2 parts of kaolin (hydrated aluminum silicate), and 1 part of a copolymer between a crosslinked methylpolysiloxane and a methylsiloxane reticular polymer (trade name "KSP-100" supplied by Shin-Etsu Chemical Co., Ltd.); and further dispersing the mixture with a homomixer. Independently, a molten mixture was prepared by stirring 3 parts of a paraffin wax (trade name "Paramix 91" supplied by Nikko Rica Corporation), 3 parts of a candelilla wax (trade name "Candelilla Wax" supplied by CERARICA NODA Co., Ltd.), 4 parts of vaseline (trade name "Vaseline-P" supplied by Nikko Rica Corporation), 5 parts of a liquid paraffin (trade name "HIWHITE 22S" supplied by Nippon Oil Corporation), 2 parts of diisostearyl malate, 3 parts of cetyl octanoate, and 2 parts of a polyoxyethylene glyceryl triisostearate (trade name "EMALEX GWIS-320", supplied by Nihon Emulsion Co., Ltd.) with heating at 80°C. The above-prepared dispersion was combined with the molten mixture, stirred, neutralized with 1 part of a 10% aqueous potassium hydroxide solution, and emulsified with a homomixer. The resulting emulsion was sequentially combined with 0.5 part of a highly polymerized poly(ethylene glycol) (trade name "POLYOX WSR-301" supplied by Union Carbide Corporation (The Dow Chemical Company)), 1 part of phenoxyethanol, 10 parts (4.0 parts in terms of solids content of urethane-modified acrylic polymer) of the urethane-modified acrylic polymer aqueous dispersion D prepared according to Preparation Example 4 of urethane-modified acrylic polymers, stirred, rapidly cooled to 35°C, and thereby yielded a wax-type hair cosmetic (hair wax).

(EXAMPLE 11)

Initially, a dispersion was prepared by dissolving a proper amount of trisodium edetate in 44.5 parts of ion-exchanged water; gradually adding and dispersing 0.2 part of a carboxyvinyl polymer (trade name "HIVISWAKO 105" supplied by Wako Pure Chemical Industries, Ltd.) in the mixture to give a homogeneous dispersion; and further adding 10.0 parts of propylene glycol and 2.0 parts of an 2-alkyl-N-carboxymethyl-N-hydroxyethylimidazolinium betaine (trade name "Ovazolin 662N" supplied by Toho Chemical Industry Co., Ltd.). In dependently, a molten mixture was prepared by heating (80°C) and mixing 10.0 parts of liquid paraffin (trade name "HIWHITE 22S" supplied by Nippon Oil Corporation), 5.0 parts of a microcrystalline wax (trade name "Microcrystalline Wax-P" supplied by Nikko Rica Corporation), 5.0 parts of a beeswax (trade name "Koshiro Deodorized Beeswax X" supplied by Koshiro Co., Ltd.), 1.0 part of isostearic acid, 3.0 parts of cetyl octanoate, 2.0 parts of a polyoxyethylene glyceryl isostearate (trade name "EMALEX GWIS-160N" supplied by Nihon Emulsion Co., Ltd.), 2.0 parts of self-emulsifiable glyceryl monostearate, 2.0 parts of a hydrogenated polyisobutene (trade name "Deodorized Polybutene-P" supplied by Nikko Rica Corporation), and 2.0 parts of a polyoxypropylene butyl ether (average number of moles of propylene oxide repeating units in polyoxypropylene moiety: 40) (trade name "UNILUB MB-370" supplied by NOF Corporation). The molten mixture was combined with the above-prepared dispersion which had been heated to 80°C, neutralized with 0.3 part of triethanolamine, and emulsified with a homomixer. While maintaining the temperature at 80°C, the resulting emulsion was sequentially combined with 1.0 part of silicic anhydride, a proper amount of a p-hydroxybenzoic ester (trade name "Mekkins M" supplied by Ueno Fine Chemicals Industry, Ltd.), 10.0 parts (4.0 parts in terms of solids content of urethane-modified acrylic polymer) of the urethane-modified acrylic polymer aqueous dispersion C prepared according to Preparation Example 3 of urethane-modified acrylic polymers, the mixture was stirred, rapidly cooled to 35°C, and thereby yielded a wax-type hair cosmetic (hair wax).

(EXAMPLE 12)

Initially, a solution was prepared by dissolving 2 parts of an associative thickener having urethane bonds {the associative thickener described in Example 8 of JP-A No. 2000-234085 [POE distearyl ether; average number of moles of ethylene oxide repeating units in polyoxyethylene (POE) moiety: 200, ratio by mass of hydrophilic moieties to hydrophobic moieties of 18:1]}, 8 parts of propylene glycol, and 2 parts of glycerol in 49.5 parts of ion-exchanged water with stirring at 70°C. Independently, a transparent molten mixture was prepared by adding 2.5 parts of a carnauba wax (trade name "Carnauba Wax" supplied by CERARICA NODA Co., Ltd.), 2 parts of an 2-alkyl-N-carboxymethyl-N-hydroxyethylimidazolinium betaine (trade name "Ovazolin 662N" supplied by Toho Chemical Industry Co., Ltd.), and 3.5 parts of a polyoxyethylene polyoxypropylene behenyl ether (average number of moles of ethylene oxide repeating units in polyoxyethylene moiety: 15, average number of moles of propylene oxide repeating units in polyoxypropylene moiety: 1) (trade name "Pepol BEP-0115" supplied by Toho Chemical Industry Co., Ltd.) to 20 parts of ion-exchanged water; and stirring the mixture at 90°C. The above-prepared solution and molten mixture were both rapidly cooled to 35°C, mixed with each other, further combined with 10 parts (4.0 parts in terms of solids content of urethane-modified acrylic polymer) of the urethane-modified acrylic polymer aqueous dispersion B prepared according to Preparation Example 2 of urethane-modified acrylic polymers and 0.5 part of phenoxyethanol, the mixture was stirred, and thereby yielded a transparent wax-type hair cosmetic (hair wax).

The hair waxes according to Examples 10 to 12 have superior hair style retentivity and impart a superior touch to the set hair, as with the hair styling gels according to Examples 5 to 9. Specifically, these hair waxes, when used for hair styling of scalp hairs, enable hair styling of scalp hairs with a natural feeling and a pliable and smooth touch and can maintain the set hair style for a long time even in an atmosphere of high humidity.

(EXAMPLE 13)

Initially, an emulsion component was prepared in the following manner. A solution was prepared by adding 2.0 parts of a high-molecular-weight polysiloxane (trade name "TSE 200A" supplied by GE Toshiba Silicone Co., Ltd.) and 0.5 part of an amino-modified high-molecular-weight silicone {the amino-modified high-molecular-weight silicone "HO-Si(CH3)(CH3)-O-[Si(CH3)(CH3)-O]3000-[Si(CH3)((CH2)3N(CH3)2)-O]6-Si(CH3)(CH3)-OH" described in Example 4 of JP-A No. Hei 5-85918} to a mixture of 5.0 parts of dimethylpolysiloxane [trade name "Silicone SH 200C-20cs" supplied by Dow Corning Toray Co., Ltd.; viscosity (25°C): 20 mPa.s] and 5.0 parts of isohexadecane (trade name "Isohexadecane" supplied by Nihon Koken Kogyo Co., Ltd.) with stirring. Independently, a mixture was prepared by mixing 3.0 parts of 1,3-butylene glycol, 2.0 parts of a polyoxyethylene cetyl ether (average number of moles of ethylene oxide repeating units in polyoxyethylene moiety: 20) (trade name "EMALEX-120" supplied by Nihon Emulsion Co., Ltd.), and 10 parts of ion-exchanged water. This mixture was combined with the above-prepared solution and emulsified with a homomixer to give the emulsion component.

Independently, an aqueous component was prepared by adding 10.0 parts (4.0 parts in terms of solids content of urethane-modified acrylic polymer) of the urethane-modified acrylic polymer aqueous dispersion D, prepared according to Preparation Example 4 of urethane-modified acrylic polymers, to 54.1 parts of ion-exchanged water.

Additionally, a solution was prepared by adding 0.2 part of a polyoxyethylene/polyoxypropylene decyl ether (average number of moles of ethylene oxide repeating units in polyoxyethylene moiety: 12, average number of moles of propylene oxide repeating units in polyoxypropylene moiety: 2) (trade name "EMALEX DAPE-0212" supplied by Nihon Emulsion Co., Ltd.), 0.1 part of behenyltrimethylammonium chloride (trade name "Catinal BTC-80" supplied by Toho Chemical Industry Co., Ltd.), 0.1 part of phenoxyethanol, and a proper amount of a perfume to 8.0 parts of ethanol with stirring. This solution was added to the above-prepared aqueous component, and the resulting mixture was further combined with the emulsion component, mixed homogenously, and thereby yielded a hair cosmetic stock. An aerosol can (capacity: 167 ml) was charged with 90 parts by mass of the stock, equipped with a stopper valve, and filled with 10 parts by mass of liquid petroleum gas (LPG), to thereby yield a mousse-type hair cosmetic (hair styling mousse).

(EXAMPLE 14)

Initially, an emulsion component was prepared in the following manner. A solution was prepared by adding 8.0 parts of a product under the trade name "Yukaformer 301" (supplied by Mitsubishi Chemical Corporation; N-methacryloyloxyethyl-N,N-dimethylammonium-$\alpha$-N-methylcarboxybetaine/alkyl methacrylate copolymer solution; content of active ingredient: 30 percent by mass) to a mixture of 5.0 parts of a dimethylpolysiloxane [trade name "Silicone SH 200C-20cs" supplied by Dow Corning Toray Co., Ltd.; viscosity (25°C): 20 mPa.s] and 5.0 parts of isohexadecane (trade name "Isohexadecane" supplied by Nihon Koken Kogyo Co., Ltd.) with stirring. Independently, a mixture was prepared by mixing 3.0 parts of 1,3-butylene glycol, 2.0 parts of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.), and 10 parts of ion-exchanged water. This mixture was combined with the above-prepared solution and emulsified with a homomixer to give the emulsion component.

Independently, an aqueous component was prepared by adding 15.0 parts (6.0 parts in terms of solids content of urethane-modified acrylic polymer) of the urethane-modified acrylic polymer aqueous dispersion C, prepared according to Preparation Example 3 of urethane-modified acrylic polymer, to 43.6 parts of ion-exchanged water.

Additionally, a solution was prepared by adding 0.2 part of lauroyl monoethanolamide, 0.1 part of behenyltrimethylammonium chloride (trade name "Catinal BTC-80" supplied by Toho Chemical Industry Co., Ltd.), 0.1 part of a paraben, and a proper amount of a perfume to 8.0 parts of ethanol with stirring. The solution was combined with the above-prepared aqueous component, further combined with the above-prepared emulsion component, mixed homogenously, and thereby yielded a hair cosmetic stock. An aerosol can (capacity: 167 ml) was charged with 90 parts by mass of the stock, equipped with a stopper valve, and filled with 10 parts by mass of liquid petroleum gas (LPG), to thereby yield a mousse-type hair cosmetic (hair styling mousse).

(EXAMPLE 15)

Initially, an emulsion component was prepared in the following manner. A solution was prepared by adding 8.5 parts of a high-molecular-weight polysiloxane (trade name "TSE 200A" supplied by GE Toshiba Silicone Co., Ltd.) to a mixture of 5.0 parts of a dimethylpolysiloxane [trade name "Silicone SH 200C-20cs" supplied by Dow Corning Toray Co., Ltd.; viscosity (25°C): 20 mPa.s] and 5.0 parts of isohexadecane (trade name "Isohexadecane" supplied by Nihon Koken Kogyo Co., Ltd.) with stirring. Independently, a mixture was prepared by mixing 3.0 parts of propylene glycol, 2.0 parts of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.), and 10 parts of ion-exchanged water. This mixture was combined with the above-prepared solution and emulsified with a homomixer to give the emulsion component.

Independently, an aqueous component was prepared by adding 10.0 parts (4.0 parts in terms of solids content of urethane-modified acrylic polymer) of the urethane-modified acrylic polymer aqueous dispersion B prepared according to Preparation Example 2 of urethane-modified acrylic polymers to 48.1 parts of ion-exchanged water.

Additionally, a solution was prepared by adding 0.2 part of lauroyl diethanolamide, 0.1 part of behenyltrimethylammonium chloride (trade name "Catinal BTC-80" supplied by Toho Chemical Industry Co., Ltd.), 0.1 part of a paraben, and a proper amount of a perfume to 8.0 parts of ethanol with stirring. The solution was combined with the above-prepared aqueous component, further combined with the above-prepared emulsion component, mixed homogenously, and thereby yielded a hair cosmetic stock. An aerosol can (capacity: 167 ml) was charged with 90 parts by mass of the stock, equipped with a stopper valve, and filled with 10 parts by mass of liquid petroleum gas (LPG), to thereby yield a mousse-type hair cosmetic (hair styling mousse).

(EXAMPLE 16)

Initially, an emulsion component was prepared in the following manner. A solution was prepared by adding 3.5

parts of an amino-modified high-molecular-weight silicone {the amino-modified high-molecular-weight silicone" (CH3) 3-Si-O-[Si(CH3)(CH3)-O]15000-[Si(CH3)((CH2)3N(CH3)(CH2)2N(CH3)C=O(C2H5))-O]4-Si-(CH3)3" described in Example 7 of JP-A No. Hei 5-85918} to a mixture of 5.0 parts of a dimethylpolysiloxane [trade name "Silicone SH 200C-20cs" supplied by Dow Corning Toray Co., Ltd.; viscosity (25°C): 20 mPa.s] and 5.0 parts of isohexadecane (trade name "Isohexadecane" supplied by Nihon Koken Kogyo Co., Ltd.) with stirring. Independently, a mixture was prepared by mixing 3.0 parts of 1,3-butylene glycol, 2.0 parts of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.), and 10 parts of ion-exchanged water. This mixture was combined with the above-prepared solution and emulsified with a homomixer to give the emulsion component.

Independently, an aqueous component was prepared by adding 12.0 parts (4.8 parts in terms of solids content of urethane-modified acrylic polymer) of the urethane-modified acrylic polymer aqueous dispersion A, prepared according to Preparation Example 1 of urethane-modified acrylic polymers, to 51.1 parts of ion-exchanged water.

Additionally, a solution was prepared by adding 0.2 part of lauric diethanolamide, 0.1 part of behenyltrimethylammonium chloride (trade name "Catinal BTC-80" supplied by Toho Chemical Industry Co., Ltd.), 0.1 part of a paraben, and a proper amount of a perfume to 8.0 parts of ethanol with stirring. The solution was combined with the aqueous component, further combined with the emulsion component, mixed homogenously, and thereby yielded a hair cosmetic stock. An aerosol can (capacity: 167 ml) was charged with 90 parts by mass of the stock, equipped with a stopper valve, and filled with 10 parts by mass of liquid petroleum gas (LPG), to thereby yield a mousse-type hair cosmetic (hair styling mousse).

(EXAMPLE 17)

Initially, an emulsion component was prepared in the following manner. A solution was prepared with agitation by adding 5.5 parts of a product under the trade name "Yukaformer AM75SM" (supplied by Mitsubishi Chemical Corporation; a betainized dialkylaminoalkyl acrylate copolymer) to a mixture of 5.0 parts of a dimethylpolysiloxane [trade name "Silicone SH 200C-20cs" supplied by Dow Corning Toray Co., Ltd.; viscosity (25°C): 20 mPa.s] and 5.0 parts of isohexadecane (trade name "Isohexadecane" supplied by Nihon Koken Kogyo Co., Ltd.). Independently, a mixture was prepared by mixing 3.0 parts of 1,3-butylene glycol, 2.0 parts of a polyoxyethylene hydrogenated caster oil (average number of moles of ethylene oxide repeating units in polyoxyethylene moiety: 40) (trade name "EMALEX HC-40" supplied by Nihon Emulsion Co., Ltd.), and 10 parts of ion-exchanged water. This mixture was combined with the above-prepared solution and emulsified with a homomixer to give the emulsion component.

Independently, an aqueous component was prepared by adding 10.7 parts (4.28 parts in terms of solids content of urethane-modified acrylic polymer) of the urethane-modified acrylic polymer aqueous dispersion D, prepared according to Preparation Example 4 of urethane-modified acrylic polymers, to 50.4 parts of ion-exchanged water.

Additionally, a solution was prepared by adding 0.2 part of a polyoxyethylene cetyl ether (average number of moles of ethylene oxide repeating units in polyoxyethylene moiety: 20) (trade name "EMALEX-120" supplied by Nihon Emulsion Co., Ltd.), 0.1 part of behenyltrimethylammonium chloride (trade name "Catinal BTC-80" supplied by Toho Chemical Industry Co., Ltd.), 0.1 part of a paraben, and a proper amount of a perfume to 8.0 parts of ethanol with stirring. The solution was combined with the aqueous component, further combined with the emulsion component, mixed homogenously, and thereby yielded a hair cosmetic stock. An aerosol can (capacity: 167 ml) was charged with 90 parts by mass of the stock, equipped with a stopper valve, and filled with 10 parts by mass of liquid petroleum gas (LPG), to thereby yield a mousse-type hair cosmetic (hair styling mousse).

The hair styling mousses according to Examples 13 to 17 each have superior hair style retentivity and can impart a superior touch to the set hair, as with the hair styling gels according to Examples 5 to 9 and the hair waxes according to Examples 10 to 12. Specifically, these hair styling mousses, when used for hair styling of scalp hairs, enable hair styling of scalp hairs with a natural feeling and a pliable and smooth touch and can maintain the set hair style for a long time even in an atmosphere of high humidity.

(EXAMPLE 18)

A hair cosmetic stock was prepared by mixing 10.0 parts (4.0 parts in terms of solids content of urethane-modified acrylic polymer) of the urethane-modified acrylic polymer aqueous dispersion D prepared according to Preparation Example 4 of urethane-modified acrylic polymers, 0.5 part of octyl palmitate, 30.0 parts of ethanol, 59.5 parts of ion-exchanged water, and a proper amount of a perfume. The stock (50 parts by mass) and dimethyl ether (50 parts by mass) were charged into an aerosol can (capacity: 167 ml) and thereby yielded a spray-type hair cosmetic (hair styling spray; aerosol spray).

(EXAMPLE 19)

A hair cosmetic stock was prepared by the procedure of Example 18. To 99.33 parts by mass of the stock was added 0.67 part by mass of nitrogen gas, and the mixture was charged into an aerosol can (capacity: 167 ml) and thereby yielded a spray-type hair cosmetic (hair styling spray; aerosol spray) using no combustible gas.

(EXAMPLE 20)

A major component was prepared by mixing 5.0 parts (4.0 parts in terms of solids content of urethane-modified acrylic polymer) of the urethane-modified acrylic polymer aqueous dispersion D prepared according to Preparation Example 4 of urethane-modified acrylic polymers, 1.0 part of a polyether-modified dimethylpolysiloxane (trade name "SILWET 10-E" supplied by Nippon Unicar Co., Ltd.), 1.0 part of glycerol, and 59.5 parts of ion-exchanged water with stirring at room temperature.

Independently, a mixture was prepared by homogeneously mixing 10.0 parts of ethanol, 0.5 part of a polyoxyethylene octyldodecyl ether (average number of moles of ethylene oxide repeating units in polyoxyethylene moiety: 20) (trade name "EMALEX OD-20" supplied by Nihon Emulsion Co., Ltd.), 0.5 part of ethylhexyl methoxycinnamate, 0.1 part of t-butylmethoxydibenzoylmethane, and a proper amount of a perfume. The mixture was added to the major component and thereby yielded a hair cosmetic as a transparent solution (hair styling spray; trigger spray).

The hair styling sprays according to Examples 18 to 20 each have superior hair style retentivity and can impart a superior touch to the set hair, as with the hair styling gels according to Examples 5 to 9, the hair waxes according to Examples 10 to 12, and the hair styling mousses according to Examples 13 to 17. Specifically, these hair styling sprays, when used for hair styling of scalp hairs, enable hair styling of scalp hairs with a natural feeling and a pliable and smooth touch and can maintain the set hair style for a long time even in an atmosphere of high humidity.

(EXAMPLE 21)

A major component was prepared by heating 44.9 parts of ion-exchanged water to 80°C; dissolving 0.5 part of a poly(vinyl alcohol) (trade name "P.V.A EG-25" supplied by Nippon Synthetic Chemical Industry Co., Ltd.) in the heated ion-exchanged water with stirring; sequentially adding thereto 5.0 parts of dipropylene glycol, 15.0 parts of a sucrose fatty acid ester (trade name "DK Ester S-160N" supplied by Daiichi Kogyo Seiyaku Co., Ltd.), 1.0 part of titanium oxide on mica (trade name "Trimiron Starluster MP-115" supplied by Merck Ltd., Japan), 0.1 part of sodium hydrogen carbonate, 0.1 part of DL-α-tocopherol acetate, a proper amount of a p-hydroxybenzoic ester (trade name "Mekkins M" supplied by Ueno Fine Chemicals Industry, Ltd.), a proper amount of sodium dehydroacetate, 10.0 parts of a black iron oxide (trade name "TAROX Synthetic Iron Oxide BL-100" supplied by Titan Kogyo Kabushiki Kaisha), 0.1 part of magnesium aluminum silicate, 0.5 part of silicic anhydride, and 0.1 part of titanium oxide; and homogenously dispersing these ingredients.

Independently, a mixture was prepared by mixing 6.0 parts of a microcrystalline wax (trade name "Microcrystalline Wax-P" supplied by Nikko Rica Corporation), 1.0 part of batyl alcohol, 3.0 parts of isostearic acid, 1.0 part of stearic acid, 0.1 part of di(phytostearyl/2-octyldodecyl) N-lauroyl-L-glutamate, 1.0 part of sorbitan monostearate, and 1.0 part of a polyoxyethylene sorbitan monostearate (average number of moles of ethylene oxide repeating units in polyoxyethylene moiety: 20). This mixture was combined with the above-prepared major component, neutralized with 0.5 part of a 10% aqueous potassium hydroxide solution, emulsified with a homomixer, sequentially combined with 1.0 part of a dimethylpolysiloxane [trade name "Silicone SH 200C-20cs" supplied by Dow Corning Toray Co., Ltd.; viscosity (25°C): 20 mPa.s], 3.0 parts of isopropanol, a proper amount of phenoxyethanol, 0.1 part of a seaweed extract (trade name "Rh. Delesseria WP" supplied by CODIF), and 5.0 parts (2.0 parts in terms of solids content of urethane-modified acrylic polymer) of the urethane-modified acrylic polymer aqueous dispersion D prepared according to Preparation Example 4 of urethane-modified acrylic polymers. The resulting mixture was homogenized by stirring, cooled to 35°C, and thereby yielded a mascara as a hair cosmetic.

(EXAMPLE 22)

Initially, a homogeneous mixture was prepared by mixing and stirring 8.0 parts of a light isoparaffin (trade name "Isopar H" supplied by Exxon-Mobil Corporation), 3.0 parts of a dimethylpolysiloxane [trade name "Silicone SH 200C-20cs" supplied by Dow Corning Toray Co., Ltd.; viscosity (25°C): 20 mPa.s], 10.0 parts of decamethylcyclopentanesiloxane (trade name "SH 245S" supplied by Dow Corning Toray Co., Ltd.), 1.0 part of trimethylsiloxysilicic acid, 2.0 parts of a poly(ethylene glycol) dioleate (trade name "WOGEL 18D" supplied by Matsumoto Fine Chemical Co., Ltd.), 2.0 parts of diglyceryl diisostearate, and 4.0 parts of a hydrogenated polyisobutene (trade name "Deodorized Polybutene-P" supplied by Nikko Rica Corporation). Independently, a dispersion was prepared by dissolving 7.0 parts (2.8 parts in

terms of solids content of urethane-modified acrylic polymer) of the urethane-modified acrylic polymer aqueous dispersion D prepared according to Preparation Example 3 of urethane-modified acrylic polymer, a proper amount of a methylpolysiloxane emulsion, 4.0 parts of 1,3-butylene glycol, 0.1 part of sodium hydrogen carbonate, a proper amount of sodium metaphosphate, 0.1 part of DL-$\alpha$-tocopherol acetate, a proper amount of a p-hydroxybenzoic ester (trade name "Mekkins M" supplied by Ueno Fine Chemicals Industry, Ltd.), and a proper amount of sodium dehydroacetate in 42.7 parts of ion-exchanged water with stirring; further adding 7.0 parts of a black iron oxide (trade name "TAROX Synthetic Iron Oxide BL-100" supplied by Titan Kogyo Kabushiki Kaisha), 0.1 part of a seaweed extract (trade name "Rh. Delesseria WP" supplied by CODIF), 1.0 part of bentonite, 2.0 parts of dimethyldistearylammonium-modified hectorite (trade name "BENTONE 38" supplied by ELEMENTIS SPECIALTIES, Inc.), 3.0 parts of a poly(vinyl acetate) emulsion (trade name "Vinylblan S-40" supplied by Nisshin Chemical Industry Co., Ltd.), and 3.0 parts of a nylon fiber (trade name "Nylon Fiber" supplied by Tsubaki Sangyo Kabushiki Kaisha, diameter: 1 to 2 mm); and dispersing these ingredients using a dispersion. The resulting dispersion was gradually added to and emulsified with the above-prepared homogeneous mixture using a homomixer and thereby yielded a mascara as a hair cosmetic.

The mascaras according to Examples 21 and 22 each have superior hair style retentivity and can impart a superior touch to the set cilia. Specifically, these mascaras, when used for styling of cilia, enable styling of the cilia with a natural feeling and a pliable and smooth touch and can retain the set style of cilia for a long time even in an atmosphere of high humidity.

Industrial Applicability

According to the present invention, there are provided hair cosmetics in response to consumers demands. Specifically, when an importance is placed on the hair styling capability, there are provided hair cosmetics that exhibit furthermore superior hair styling capability and furthermore superior hair style retentivity while maintaining natural feeling at certain level. On the other hand, when an importance is placed on the smooth finish, there are provided hair cosmetics that exhibit satisfactory natural finishing capability and smooth touch with no or substantially no coarse/stiff feeling while maintaining hair style retentivity at certain level.

## Claims

1. A hair cosmetic comprising a urethane-modified acrylic polymer having a structure in which a urethane polymer chain is bonded to an acrylic polymer chain through a linkage segment containing a silicon-oxygen bond,
wherein the acrylic polymer chain is a residue of an acrylic polymer derived at least from an acrylic monomer (A) and one or both of a compound (B1) and a compound (B2), where the compound (B1) has a hydrolyzable silicon-containing group and an ethylenically unsaturated bond-containing group, and the compound (B2) has a hydrolyzable silicon-containing group and a mercapto group; and
wherein the urethane polymer chain is a residue of a urethane polymer (C) having a hydrolyzable silicon-containing group.

2. The hair cosmetic according to claim 1, wherein the urethane polymer (C) having a hydrolyzable silicon-containing group is an alkoxysililated urethane polymer (C1) having a hydrophilic group.

3. The hair cosmetic according to claim 2, wherein the alkoxysililated urethane polymer (C1) having a hydrophilic group is a terminally alkoxysililated urethane polymer having a hydrophilic group and corresponds to a urethane polymer having a hydrophilic group, at least part of terminal isocyanato groups of the urethane polymer being alkoxysililated.

4. The hair cosmetic according to one of claims 2 and 3, wherein the alkoxysililated urethane polymer (C1) having a hydrophilic group is a urethane polymer having a hydrophilic group and a terminal alkoxysilyl group and is a reaction product among following Components (C1-a), (C1-b), (C1-c), and (C1-d):

    (C1-a): a compound having two or more active-hydrogen-containing groups but having no hydrophilic group;
    (C1-b): a compound having a hydrophilic group and two or more active-hydrogen-containing groups;
    (C1-c): a polyisocyanate compound; and
    (C1-d): an alkoxysilane compound having an active-hydrogen-containing group.

5. The hair cosmetic according to claim 4, wherein the alkoxysilane compound (C1-d) having an active-hydrogen-containing group is an amino-containing alkoxysilane compound (C1-d1) that contains an amino group as the active-hydrogen-containing group.

6. The hair cosmetic according to any one of claims 1 to 5, wherein the linkage segment is a silicone polymer chain.

7. The hair cosmetic according to claim 6, wherein the silicone polymer chain comprises a hydrolyzable silicon-containing group and an ethylenically unsaturated bond-containing group of the compound (B1) and/or a hydrolyzable silicon-containing group and a mercapto group of the compound (B2); a hydrolyzable silicon-containing group of the urethane polymer (C); and a silane compound (D) having a hydrolyzable silicon-containing group.

8. The hair cosmetic according to claim 7, wherein the silane compound (D) having a hydrolyzable silicon-containing group is an alkoxy-containing silane compound (D1).

9. The hair cosmetic according to claim 8, wherein the alkoxy-containing silane compound (D1) comprises at least a dialkoxysilyl-containing silane compound (D1-1).

10. The hair cosmetic according to claim 8, wherein the alkoxy-containing silane compound (D1) comprises at least a trialkoxysilyl-containing silane compound (D1-2).

11. The hair cosmetic according to any one of claims 8 to 10, wherein the alkoxy-containing silane compound (D1) comprises both a dialkoxysilyl-containing silane compound (D1-1) and a trialkoxysilyl-containing silane compound (D1-2).

12. The hair cosmetic according to any one of claims 1 to 14, which comprises 0.1 to 10 percent by mass of the urethane-modified acrylic polymer.

## INTERNATIONAL SEARCH REPORT

International application No.
PCT/JP2007/063106

A. CLASSIFICATION OF SUBJECT MATTER
*A61K8/898*(2006.01)i, *A61K8/899*(2006.01)i, *A61Q5/00*(2006.01)i, *A61Q5/06*(2006.01)i, *C08F290/06*(2006.01)i, *C08G18/65*(2006.01)i

According to International Patent Classification (IPC) or to both national classification and IPC

B. FIELDS SEARCHED

Minimum documentation searched (classification system followed by classification symbols)
A61K8/898, A61K8/899, A61Q5/00, A61Q5/06, C08F290/06, C08G18/65

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAPlus (STN)

C. DOCUMENTS CONSIDERED TO BE RELEVANT

| Category* | Citation of document, with indication, where appropriate, of the relevant passages | Relevant to claim No. |
|---|---|---|
| X | WO 2005/054341 A1 (Konishi Co., Ltd.), 16 June, 2005 (16.06.05), Full text & CN 1914252 A & KR 20060103940 A & US 2007/117902 A1 | 1-12 |
| A | JP 2001-323040 A (Auto Chemical Ind. Co., Ltd.), 20 November, 2001 (20.11.01), (Family: none) | 1-12 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 September, 2007 (19.09.07) | 02 October, 2007 (02.10.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/063106

C (Continuation). DOCUMENTS CONSIDERED TO BE RELEVANT

| Category* | Citation of document, with indication, where appropriate, of the relevant passages | Relevant to claim No. |
|---|---|---|
| A | WO 00/12588 A1 (BASF AG.), 09 March, 2000 (09.03.00), & AT 325146 T & CN 1145660 C & DE 19838852 A1 & DE 19923276 A1 & DE 59913390 D1 & EP 1117726 A1 & ES 2264268 T & JP 2002-523582 A & US 6524564 B1 | 1-12 |
| A | WO 03/011937 A1 (3M INNOVATIVE PROPERTIES CO.), 13 February, 2003 (13.02.03), & BR 0116773 A & CA 2434246 A1 & CN 1211411 C & EP 1362072 A1 & JP 2004-521957 A & MX PA03006295 A & US 2002/146382 A1 | 1-12 |
| P,A | WO 2006/073174 A1 (Shiseido Co., Ltd.), 13 July, 2006 (13.07.06), & JP 2006-213706 A | 1-12 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005054341 A
- WO 2005054341 A1
- JP 2003171244 A
- JP 2000072626 A
- JP 3670841 B
- JP HEI892044 A
- JP 2003171236 A
- JP HEI585918 A
- JP 2000234085 A